# EUROPEAN PATENT APPLICATION

(11) **EP 4 450 641 A2**
(43) Date of publication of application: **23.10.2024**
(21) Application number: 24194076.6
(22) Date of filing: 07.01.2022
(51) Int. Cl.: C12Q 1/6841

(54) **METHOD FOR TRANSPOSASE MEDIATED SPATIAL TAGGING AND ANALYZING GENOMIC DNA IN A BIOLOGICAL SAMPLE**

(30) Priority: 29.01.2021 US 202163143438 P; 26.03.2021 US 202163166708 P
(62) Divisional of application: 22702549.1
(71) Applicant: 10x Genomics, Inc., Pleasanton, CA 94588-3260 (US)
(72) Inventor: STAHL, Patrik, 114 33 Stockholm (SE); MARKLUND, Maja, 114 33 Stockholm (SE); LLORENS BOBADILLA, Enric, 114 33 Stockholm (SE); FRISEN, Jonas, 114 33 Stockholm (SE)
(74) Representative: Vossius & Partner Patentanwälte Rechtsanwälte mbB

(57) **Abstract**

The present disclosure relates to materials and methods for spatially analyzing nucleic acids fragmented with a transposase enzyme in a biological sample.

## Description

### CROSS-REFERENCE TO RELATED APPLICATIONS

This application claims priority to U.S. Provisional Patent Application No. 63/143,438, filed on January 29, 2021 and U.S. Provisional Patent Application No. 63/166,708, filed on March 26, 2021. The contents of these applications are incorporated herein by reference in their entireties.

### BACKGROUND

Cells within a tissue have differences in cell morphology and/or function due to varied analyte levels (e.g., gene and/or protein expression) within the different cells. The specific position of a cell within a tissue (e.g., the cell's position relative to neighboring cells or the cell's position relative to the tissue microenvironment) can affect, e.g., the cell's morphology, differentiation, fate, viability, proliferation, behavior, signaling, and cross-talk with other cells in the tissue.

Spatial heterogeneity has been previously studied using techniques that typically provide data for a handful of analytes in the context of intact tissue or a portion of a tissue (e.g., tissue section), or provide significant analyte data from individual, single cells, but fails to provide information regarding the position of the single cells from the originating biological sample (e.g., tissue).

Chromatin structure can be different between cells in a biological sample or between biological samples from the same tissue. Assaying differences in accessible chromatin can be indicative of transcriptionally active sequences, e.g., genes, in a particular cell. Further understanding the transcriptionally active regions within chromatin will enable identification of which genes contribute to a cell's function and/or phenotype.

### SUMMARY

The present disclosure generally describes methods for spatially analyzing genomic DNA present in a biological sample.

Methods have been developed to study epigenomes, e.g., chromatin accessibility assays (ATAC-seq) or identifying proteins associated with chromatin e.g., (ChIP-seq). These assays help identify regulators (e.g., cis regulators and/or trans regulators) that contribute to dynamic cellular phenotypes. While ATAC-Seq and ChIP-Seq have been invaluable in defining epigenetic variability within a cell population, conventional applications of these methods are limited in their ability to spatially resolve the associated genes that promote cellular variation. Spatial methods are already known, however, additional and/or alternative methods are still needed.

Thus, the present disclosure relates generally to the spatial tagging and analysis of nucleic acids. In some embodiments, provided herein are methods that utilize a transposome to fragment genomic DNA (e.g., open chromatin, accessible chromatin) and to capture the fragmented DNA on a spatial array, thus revealing epigenomic insights regarding the structural features contributing to cellular regulation within the spatial context of a biological sample.

Provided herein are methods for determining genomic DNA accessibility, the method including: (a) a biological sample on an array including a plurality of capture probes, where a capture probe of the plurality of capture probes includes: (i) a spatial barcode and (ii) a capture domain; (b) contacting a plurality of splint oligonucleotides to the biological sample, where a splint oligonucleotide hybridizes to the capture domain; (c) contacting a transposome to the biological sample to insert transposon end sequences into accessible genomic DNA, thereby generating fragmented genomic DNA; (d) hybridizing the fragmented genomic DNA to the splint oligonucleotide and ligating the fragmented genomic DNA to the capture probe; (e) releasing one or more non-ligated transposon end sequences from the ligated fragmented genomic DNA; and (f) determining (i) a sequence of the spatial barcode or a complement thereof, and (ii) all or a portion of a sequence of the fragmented genomic DNA, or a complement thereof, and using the determined sequences of (i) and (ii) to determine genomic DNA accessibility in the biological sample.

In some embodiments, the array includes one or more features. In some embodiments, the one or more features includes a bead.

In some embodiments, the capture probe further includes a cleavage domain, one or more functional domains, a unique molecular identifier, or combinations thereof.

In some embodiments, the method includes an active migration step where the fragmented genomic DNA is migrated to the array by applying an electric field.

In some embodiments, the hybridizing in step (b) includes hybridizing the splint oligonucleotide, or a portion thereof, to the capture domain, or a portion thereof, of the capture probe. In some embodiments, the hybridizing in step (d) includes hybridizing the splint oligonucleotide, or a portion thereof, to a transposon end sequence or a portion thereof, of a fragmented genomic DNA.

In some embodiments, the ligating is performed using a DNA ligase.

In some embodiments, the method includes extending a 3' end of the capture probe using the fragmented genomic DNA as a template. In some embodiments, the extending step is performed using a DNA polymerase having strand displacement activity.

In some embodiments, the method includes performing gap filling between the splint oligonucleotide and the fragmented genomic DNA.

In some embodiments, the transposome includes a transposase enzyme, and where the transposase enzyme is a Tn5 transposase enzyme, a Mu transposase enzyme, a Tn7 transposase enzyme, a *Vibrio* species transposase, or functional derivatives thereof. In some embodiments, the Tn5 transposase enzyme includes a sequence that is at least 80% identical to SEQ ID NO: 1.

In some embodiments, the determining in step (f) includes sequencing (i) the spatial barcode or a complement thereof, and (ii) all or a portion of the sequence of the fragmented genomic DNA or a complement thereof and further determining the location of the accessible genomic DNA in the biological sample.

In some embodiments, the method includes imaging the biological sample before or after contacting the biological sample with the array.

In some embodiments, the releasing in step (d) includes heating the biological sample. In some embodiments, the heating includes heating to a temperature of about 65°C to 85°C. In some embodiments, the heating includes heating to a temperature of about 65°C to about 80°C. In some embodiments, the heating includes heating to a temperature of about 75°C.

In some embodiments, the method includes staining the biological sample. In some embodiments, the staining includes hematoxylin and eosin staining.

In some embodiments, contacting the transposome to the biological sample is performed under a chemical permeabilization condition, under an enzymatic permeabilization condition, or both. In some embodiments, the chemical permeabilization condition includes a detergent. In some embodiments, the detergent is one or more of NP-40, Tween-20, Triton X-100, and Digitonin. In some embodiments, the detergent is at a concentration from about 0.001% (v/v) to about 1.0% (v/v).

In some embodiments, contacting the transposome to the biological sample is performed after an enzymatic pre-permeabilization condition. In some embodiments, the enzymatic pre-permeabilization condition includes a protease. In some embodiments, the protease is a pepsin, a collagenase, a Proteinase K, and combinations thereof. In some embodiments, the protease is collagenase.

Also provided herein are methods for determining genomic DNA accessibility, the method including: (a) a biological sample on an array including a plurality of capture probes, where a capture probe of the plurality of capture probes includes: (i) a spatial barcode and (ii) a capture domain; (b) contacting a transposome to the biological sample to insert transposon end sequences into accessible genomic DNA, thereby generating fragmented genomic DNA; (c) hybridizing a transposon end sequence of the fragmented genomic DNA to the capture domain of the capture probe; (d) releasing transposon end sequences not bound to the capture domain; and (e) determining (i) a sequence of the spatial barcode or a complement thereof, and (ii) all or a portion of a sequence of the fragmented genomic DNA, or a complement thereof, and using the determined sequences of (i) and (ii) to determine genomic DNA accessibility in the biological sample.

In some embodiments, the array includes one or more features. In some embodiments, the one or more features includes a bead.

In some embodiments, the capture probe further includes a cleavage domain, one or more functional domains, a unique molecular identifier, or combinations thereof.

In some embodiments, the method further includes an active migration step where the fragmented genomic DNA is migrated to the array by applying an electric field.

In some embodiments, the hybridizing in step (c) includes hybridizing the transposon end sequence, or a portion thereof, to the capture domain, or a portion thereof, of the capture probe.

In some embodiments, the method includes extending a 3' end of the capture probe using the fragmented genomic DNA as a template. In some embodiments, the extending step is performed using a DNA polymerase having strand displacement activity.

In some embodiments, the method includes performing gap filling between the transposon end sequence and the fragmented genomic DNA.

In some embodiments, the transposome includes a transposase enzyme, and where the transposase enzyme is a Tn5 transposase enzyme, a Mu transposase enzyme, a Tn7 transposase enzyme, a *Vibrio* species transposase, or functional derivatives thereof. In some embodiments, the Tn5 transposase enzyme includes a sequence that is at least 80% identical to SEQ ID NO: 1.

In some embodiments, the determining in step (e) includes sequencing (i) the sequence of the spatial barcode or a complement thereof, and (ii) all or a portion of the sequence of the fragmented genomic DNA or a complement thereof and further determining the location of the accessible genomic DNA in the biological sample.

In some embodiments, the method includes imaging the biological sample before or after contacting the biological sample with the array.

In some embodiments, the releasing in step (d) includes heating the biological sample. In some embodiments, the heating includes heating to a temperature of about 65°C to 85°C. In some embodiments, the heating includes heating to a temperature of about 65°C to about 80°C. In some embodiments, the heating includes heating to a temperature of about 75°C.

In some embodiments, the method includes staining the biological sample. In some embodiments, the staining includes hematoxylin and eosin staining.

In some embodiments, contacting the transposome to the biological sample is performed after a chemical permeabilization condition, under an enzymatic permeabilization condition, or both. In some embodiments, the chemical permeabilization condition includes a detergent. In some embodiments, the detergent is one or more of NP-40, Tween-20, Triton X-100, and Digitonin. In some embodiments, the detergent is at a concentration from about 0.001% (v/v) to about 0.1% (v/v). In some embodiments, the transposome to the biological sample is performed after an enzymatic pre-permeabilization condition. In some embodiments, the enzymatic pre-permeabilization condition includes a protease. In some embodiments, the protease is a pepsin, a collagenase, a Proteinase K, and combinations thereof. In some embodiments, the protease in a collagenase.

Also provided herein are methods for determining the location of DNA in a biological sample, the method including: (a) a biological sample on an array including a plurality of capture probes, where a capture probe of the plurality of capture probes includes: (i) a spatial barcode and (ii) a capture domain; (b) contacting the biological sample with a protease, where the protease is capable of degrading one or more histone proteins, thereby releasing the DNA; (c) contacting a transposome to the biological sample to insert transposon end sequences into the released genomic DNA, thereby generated fragmented genomic DNA; (d) hybridizing a transposon end sequence of the fragmented DNA to the capture domain; (e) releasing transposon end sequences not bound to the capture domain; and (f) determining (i) a sequence of the spatial barcode or a complement thereof, and (ii) all or a portion of a sequence of the DNA, or a complement thereof, and using the determined sequences of (i) and (ii) to determine the location of DNA in the biological sample.

In some embodiments, the protease is capable of degrading at least one linker histone protein and at least one core histone protein in the biological sample. In some embodiments, the protease is capable of degrading at least one histone from each core histone family in the biological sample. In some embodiments, the protease is a serine protease, an aspartyl protease, a peptidase family C1 enzyme, a protease that is inhibited by the diazomethane inhibitor Z-Phe-Phe-CHN(2) or the epoxide inhibitor E-64, a lysosomal protease, collagenase, or an azurophilic enzyme. In some embodiments, the protease is collagenase.

In some embodiments, the capture domain includes a homopolymeric sequence. In some embodiments, the capture domain includes a unique sequence.

In some embodiments, the capture probe further includes a cleavage domain, one or more functional domain, a unique molecular identifier, or combinations thereof.

In some embodiments, the method includes an active migration step where the fragmented genomic DNA is migrated to the array by applying an electric field.

In some embodiments, the hybridizing in step (d) includes hybridizing the transposon end sequence, or a portion thereof, to the capture domain, or a portion thereof, of the capture probe.

In some embodiments, the method includes extending a 3' end of the capture probe using the fragmented genomic DNA as a template. In some embodiments, the extending step is performed using a DNA polymerase having strand displacement activity.

In some embodiments, the method includes gap filling between the transposon end sequence and the fragmented genomic DNA.

In some embodiments, the transposome includes a transposase enzyme, and where the transposase enzyme is a Tn5 transposase enzyme, a Mu transposase enzyme, a Tn7 transposase enzyme, a Vibrio species transposase, or functional derivatives thereof. In some embodiments, the Tn5 transposase enzyme comprise a sequence that is at least 80% identical to SEQ ID NO: 1.

In some embodiments, the determining in step (f) includes sequencing (i) the spatial barcode or a complement thereof, and (ii) all or a portion of the sequence of the fragmented genomic DNA or a complement thereof.

In some embodiments, the method further includes imaging and/or staining the biological sample. In some embodiments, the staining includes haematoxylin and eosin staining.

In some embodiments, the protease is contacted with the biological sample from about 5 minutes to about 15 minutes. In some embodiments, the protease is contacted with the biological sample for about 10 minutes. In some embodiments, the protease is contacted with the biological sample at a temperature from about 30°C to about 45°C. In some embodiments, the protease is contacted with the biological sample at a temperature of about 37°C.

In some embodiments, the releasing in step (d) includes heating the biological sample. In some embodiments, the heating includes heating to a temperature of about 65°C to 85°C.

In some embodiments, determining the location of DNA in a biological sample further includes spatially analyzing the whole genome of the biological sample.

In some embodiments, the biological sample is a tissue section. In some embodiments, the tissue section is a fresh, frozen tissue section. In some embodiments, the tissue section is a fixed tissue section. In some embodiments, the fixed tissue section is a formalin-fixed paraffin-embedded fixed tissue section, an acetone fixed tissue section, a paraformaldehyde fixed tissue section, or a methanol fixed tissue section.

All publications, patents, and patent applications mentioned in this specification are herein incorporated by reference to the same extent as if each individual publication, patent, patent application, or item of information was specifically and individually indicated to be incorporated by reference. To the extent publications, patents, patent applications, and items of information incorporated by reference contradict the disclosure contained in the specification, the specification is intended to supersede and/or take precedence over any such contradictory material.

Where values are described in terms of ranges, it should be understood that the description includes the disclosure of all possible sub-ranges within such ranges, as well as specific numerical values that fall within such ranges irrespective of whether a specific numerical value or specific sub-range is expressly stated.

The term "each," when used in reference to a collection of items, is intended to identify an individual item in the collection but does not necessarily refer to every item in the collection, unless expressly stated otherwise, or unless the context of the usage clearly indicates otherwise.

Various embodiments of the features of this disclosure are described herein. However, it should be understood that such embodiments are provided merely by way of example, and numerous variations, changes, and substitutions can occur to those skilled in the art without departing from the scope of this disclosure. It should also be understood that various alternatives to the specific embodiments described herein are also within the scope of this disclosure.

### DESCRIPTION OF DRAWINGS

The following drawings illustrate certain embodiments of the features and advantages of this disclosure. These embodiments are not intended to limit the scope of the appended claims in any manner. Like reference symbols in the drawings indicate like elements.
**FIG. 1** shows an exemplary capture probe.
**FIGs. 2** shows an exemplary spatial assay for transposase accessible chromatin (spATAC) workflow.
**FIGs. 3A-B** show **A**) hematoxylin and eosin (H&E) staining and **B**) gene expression patterns of a mouse brain with a human glioma xenograft tissue section.
**FIGs. 4A-B** show **A**) H&E staining and **B**) gene expression patterns of different species in a mouse brain tissue section to test spatial resolution of the workflow described in **FIG. 2****.**
**FIGs. 5A-B** are graphs of replicate experiments showing the number of spots (y axis) by the number of unique molecule identifiers (x axis) identified at each spot. Images corresponding to the graphs are shown above each graph.
**FIGs. 6A-B** are exemplary graphs indicating recovery of nucleosome periodicity when practicing methods described herein; bp=base pair, FU=fluorescent units.
**FIGs. 7A-H** shows two consecutive mouse tissue sections immunostained with a SOX9 antibody prior (**FIGs. 7A** and **7E**) to the spatial ATAC-seq workflow. **FIGs. 7B** and **7F** show the total numbers of tagmented DNA fragments captured per spot. **FIGs. 7C** and **7G** are graphs showing transcriptional start site (TSS) enrichment and the corresponding nucleosome periodicity when practicing the methods describe herein (**FIGs. 7D** and **7H**).
**FIG. 8** shows genome traces of ATAC-seq read densities for a reference mouse dataset (e13,5; top) and spatial ATAC-seq dataset (e13,5; middle) of the mouse embryos shown in **FIGs. 7A** and **7E****.** Spatial ATAC-seq signal enrichment and peak calling (bottom) shows matching positions for fragment enrichment.
**FIGs. 9A-B** show unbiased graph-based clustering (**FIG. 9A**) and the clusters assignment of each spot in the tissue section (**FIG. 9B**).
**FIGs. 10A-D** shows UMAP plots (**FIG. 10A** and **10C**) colored by the relative accessibility of two gene regions found to be differentially accessible between regions of the tissue section (**FIG. 10B** and **10D**).
**FIGs. 11A-F** shows in the first panel region clustering based on gene expression (**FIG**. **11A**). Spatial clusters are indicated with numbers. **FIGs. 11B-11F** show accessibility of marker genes for each cluster in an adjacent section.
**FIG. 12** shows genomic traces of spatial ATAC-seq signal enrichment showing accessibility of one such region found to be more accessible in tissue cluster 7.

### DETAILED DESCRIPTION

Spatial analysis methodologies and compositions described herein can provide a vast amount of analyte and/or expression data for a variety of analytes within a biological sample at high spatial resolution, while retaining native spatial context. Spatial analysis methods and compositions can include, e.g., the use of a capture probe including a spatial barcode (e.g., a nucleic acid sequence that provides information as to the location or position of an analyte within a cell or a tissue sample (e.g., mammalian cell or a mammalian tissue sample) and a capture domain that is capable of binding to an analyte (e.g., a protein and/or a nucleic acid) produced by and/or present in a cell. Spatial analysis methods and compositions can also include the use of a capture probe having a capture domain that captures an intermediate agent for indirect detection of an analyte. For example, the intermediate agent can include a nucleic acid sequence (e.g., a barcode) associated with the intermediate agent. Detection of the intermediate agent is therefore indicative of the analyte in the cell or tissue sample.

Non-limiting aspects of spatial analysis methodologies and compositions are described in U.S. Patent Nos. 10,774,374, 10,724,078, 10,480,022, 10,059,990, 10,041,949, 10,002,316, 9,879,313, 9,783,841, 9,727,810, 9,593,365, 8,951,726, 8,604,182, 7,709,198, U.S. Patent Application Publication Nos. 2020/239946, 2020/080136, 2020/0277663, 2020/024641, 2019/330617, 2019/264268, 2020/256867, 2020/224244, 2019/194709, 2019/161796, 2019/085383, 2019/055594, 2018/216161, 2018/051322, 2018/0245142, 2017/241911, 2017/089811, 2017/067096, 2017/029875, 2017/0016053, 2016/108458, 2015/000854, 2013/171621, WO 2018/091676, WO 2020/176788, Rodriques et al., Science 363(6434):1463-1467, 2019; Lee et al., Nat. Protoc. 10(3):442-458, 2015; Trejo et al., PLoS ONE 14(2):e0212031, 2019; Chen et al., Science 348(6233):aaa6090, 2015; Gao et al., BMC Biol. 15:50, 2017; and Gupta et al., Nature Biotechnol. 36:1197-1202, 2018; the Visium Spatial Gene Expression Reagent Kits User Guide (e.g., Rev C, dated June 2020), and/or the Visium Spatial Tissue Optimization Reagent Kits User Guide (e.g., Rev C, dated July 2020), both of which are available at the 10x Genomics Support Documentation website, and can be used herein in any combination. Further non-limiting aspects of spatial analysis methodologies and compositions are described herein.

Some general terminology that may be used in this disclosure can be found in Section (I)(b) of WO 2020/176788 and/or U.S. Patent Application Publication No. 2020/0277663. Typically, a "barcode" is a label, or identifier, that conveys or is capable of conveying information (e.g., information about an analyte in a sample, a bead, and/or a capture probe). A barcode can be part of an analyte, or independent of an analyte. A barcode can be attached to an analyte. A particular barcode can be unique relative to other barcodes. For the purpose of this disclosure, an "analyte" can include any biological substance, structure, moiety, or component to be analyzed. The term "target" can similarly refer to an analyte of interest.

Analytes can be broadly classified into one of two groups: nucleic acid analytes, and non-nucleic acid analytes. Examples of non-nucleic acid analytes include, but are not limited to, lipids, carbohydrates, peptides, proteins, glycoproteins (N-linked or O-linked), lipoproteins, phosphoproteins, specific phosphorylated or acetylated variants of proteins, amidation variants of proteins, hydroxylation variants of proteins, methylation variants of proteins, ubiquitylation variants of proteins, sulfation variants of proteins, viral proteins (e.g., viral capsid, viral envelope, viral coat, viral accessory, viral glycoproteins, viral spike, etc.), extracellular and intracellular proteins, antibodies, and antigen binding fragments. In some embodiments, the analyte(s) can be localized to subcellular location(s), including, for example, organelles, e.g., mitochondria, Golgi apparatus, endoplasmic reticulum, chloroplasts, endocytic vesicles, exocytic vesicles, vacuoles, lysosomes, etc. In some embodiments, analyte(s) can be peptides or proteins, including without limitation antibodies and enzymes. Additional examples of analytes can be found in Section (I)(c) of WO 2020/176788 and/or U.S. Patent Application Publication No. 2020/0277663. In some embodiments, an analyte can be detected indirectly, such as through detection of an intermediate agent, for example, a ligation product or an analyte capture agent (e.g., an oligonucleotide-conjugated antibody), such as those described herein.

A "biological sample" is typically obtained from the subject for analysis using any of a variety of techniques including, but not limited to, biopsy, surgery, and laser capture microscopy (LCM), and generally includes cells and/or other biological material from the subject. In some embodiments, a biological sample can be a tissue section. In some embodiments, a biological sample can be a fixed and/or stained biological sample (e.g., a fixed and/or stained tissue section). Non-limiting examples of stains include histological stains (e.g., hematoxylin and/or eosin) and immunological stains (e.g., fluorescent stains). In some embodiments, a biological sample (e.g., a fixed and/or stained biological sample) can be imaged. Biological samples are also described in Section (I)(d) of WO 2020/176788 and/or U.S. Patent Application Publication No. 2020/0277663.

In some embodiments, a biological sample is permeabilized with one or more permeabilization reagents. For example, permeabilization of a biological sample can facilitate analyte capture. Exemplary permeabilization agents and conditions are described in Section (I)(d)(ii)(13) or the Exemplary Embodiments Section of WO 2020/176788 and/or U.S. Patent Application Publication No. 2020/0277663.

Array-based spatial analysis methods involve the transfer of one or more analytes from a biological sample to an array of features on a substrate, where each feature is associated with a unique spatial location on the array. Subsequent analysis of the transferred analytes includes determining the identity of the analytes and the spatial location of the analytes within the biological sample. The spatial location of an analyte within the biological sample is determined based on the feature to which the analyte is bound (e.g., directly or indirectly) on the array, and the feature's relative spatial location within the array.

A "capture probe" refers to any molecule capable of capturing (directly or indirectly) and/or labelling an analyte (e.g., an analyte of interest) in a biological sample. In some embodiments, the capture probe is a nucleic acid or a polypeptide. In some embodiments, the capture probe includes a barcode (e.g., a spatial barcode and/or a unique molecular identifier (UMI)) and a capture domain). In some embodiments, a capture probe can include a cleavage domain and/or a functional domain (e.g., a primer-binding site, such as for next-generation sequencing (NGS)). See, e.g., Section (II)(b) (e.g., subsections (i)-(vi)) of WO 2020/176788 and/or U.S. Patent Application Publication No. 2020/0277663. Generation of capture probes can be achieved by any appropriate method, including those described in Section (II)(d)(ii) of WO 2020/176788 and/or U.S. Patent Application Publication No. 2020/0277663.

In some embodiments, more than one analyte type (e.g., nucleic acids and proteins) from a biological sample can be detected (e.g., simultaneously or sequentially) using any appropriate multiplexing technique, such as those described in Section (IV) of WO 2020/176788 and/or U.S. Patent Application Publication No. 2020/0277663.

In some embodiments, detection of one or more analytes (e.g., protein analytes) can be performed using one or more analyte capture agents. As used herein, an "analyte capture agent" refers to an agent that interacts with an analyte (e.g., an analyte in a biological sample) and with a capture probe (e.g., a capture probe attached to a substrate or a feature) to identify the analyte. In some embodiments, the analyte capture agent includes: (i) an analyte binding moiety (e.g., that binds to an analyte), for example, an antibody or antigen-binding fragment thereof; (ii) analyte binding moiety barcode; and (iii) an analyte capture sequence. As used herein, the term "analyte binding moiety barcode" refers to a barcode that is associated with or otherwise identifies the analyte binding moiety. As used herein, the term "analyte capture sequence" refers to a region or moiety configured to hybridize to, bind to, couple to, or otherwise interact with a capture domain of a capture probe. In some cases, an analyte binding moiety barcode (or portion thereof) may be able to be removed (e.g., cleaved) from the analyte capture agent. Additional description of analyte capture agents can be found in Section (II)(b)(ix) of WO 2020/176788 and/or Section (II)(b)(viii) U.S. Patent Application Publication No. 2020/0277663.

There are at least two methods to associate a spatial barcode with one or more neighboring cells, such that the spatial barcode identifies the one or more cells, and/or contents of the one or more cells, as associated with a particular spatial location. One method is to promote analytes or analyte proxies (e.g., intermediate agents) out of a cell and towards a spatially-barcoded array (e.g., including spatially-barcoded capture probes). Another method is to cleave spatially-barcoded capture probes from an array and promote the spatially-barcoded capture probes towards and/or into or onto the biological sample.

In some cases, capture probes may be configured to prime, replicate, and consequently yield optionally barcoded extension products from a template (e.g., a DNA or RNA template, such as an analyte or an intermediate agent (e.g., a ligation product or an analyte capture agent), or a portion thereof), or derivatives thereof (see, e.g., Section (II)(b)(vii) of WO 2020/176788 and/or U.S. Patent Application Publication No. 2020/0277663 regarding extended capture probes). In some cases, capture probes may be configured to form ligation products with a template (e.g., a DNA or RNA template, such as an analyte or an intermediate agent, or portion thereof), thereby creating ligations products that serve as proxies for a template.

As used herein, an "extended capture probe" refers to a capture probe having additional nucleotides added to the terminus (e.g., 3' or 5' end) of the capture probe thereby extending the overall length of the capture probe. For example, an "extended 3' end" indicates additional nucleotides were added to the most 3' nucleotide of the capture probe to extend the length of the capture probe, for example, by polymerization reactions used to extend nucleic acid molecules including templated polymerization catalyzed by a polymerase (e.g., a DNA polymerase or a reverse transcriptase). In some embodiments, extending the capture probe includes adding to a 3' end of a capture probe a nucleic acid sequence that is complementary to a nucleic acid sequence of an analyte or intermediate agent specifically bound to the capture domain of the capture probe. In some embodiments, the capture probe is extended using reverse transcription. In some embodiments, the capture probe is extended using one or more DNA polymerases. The extended capture probes include the sequence of the capture probe and the sequence of the spatial barcode of the capture probe.

In some embodiments, extended capture probes are amplified (e.g., in bulk solution or on the array) to yield quantities that are sufficient for downstream analysis, e.g., via DNA sequencing. In some embodiments, extended capture probes (e.g., DNA molecules) act as templates for an amplification reaction (e.g., a polymerase chain reaction).

Additional variants of spatial analysis methods, including in some embodiments, an imaging step, are described in Section (II)(a) of WO 2020/176788 and/or U.S. Patent Application Publication No. 2020/0277663. Analysis of captured analytes (and/or intermediate agents or portions thereof), for example, including sample removal, extension of capture probes, sequencing (e.g., of a cleaved extended capture probe and/or a cDNA molecule complementary to an extended capture probe), sequencing on the array (e.g., using, for example, in situ hybridization or in situ ligation approaches), temporal analysis, and/or proximity capture, is described in Section (II)(g) of WO 2020/176788 and/or U.S. Patent Application Publication No. 2020/0277663. Some quality control measures are described in Section (II)(h) of WO 2020/176788 and/or U.S. Patent Application Publication No. 2020/0277663.

Spatial information can provide information of biological and/or medical importance. For example, the methods and compositions described herein can allow for: identification of one or more biomarkers (e.g., diagnostic, prognostic, and/or for determination of efficacy of a treatment) of a disease or disorder; identification of a candidate drug target for treatment of a disease or disorder; identification (e.g., diagnosis) of a subject as having a disease or disorder; identification of stage and/or prognosis of a disease or disorder in a subject; identification of a subject as having an increased likelihood of developing a disease or disorder; monitoring of progression of a disease or disorder in a subject; determination of efficacy of a treatment of a disease or disorder in a subject; identification of a patient subpopulation for which a treatment is effective for a disease or disorder; modification of a treatment of a subject with a disease or disorder; selection of a subject for participation in a clinical trial; and/or selection of a treatment for a subject with a disease or disorder.

Spatial information can provide information of biological importance. For example, the methods and compositions described herein can allow for: identification of transcriptome and/or proteome expression profiles (e.g., in healthy and/or diseased tissue); identification of multiple analyte types in close proximity (e.g., nearest neighbor analysis); determination of up- and/or down-regulated genes and/or proteins in diseased tissue; characterization of tumor microenvironments; characterization of tumor immune responses; characterization of cells types and their co-localization in tissue; and identification of genetic variants within tissues (e.g., based on gene and/or protein expression profiles associated with specific disease or disorder biomarkers).

Typically, for spatial array-based methods, a substrate functions as a support for direct or indirect attachment of capture probes to features of the array. A "feature" is an entity that acts as a support or repository for various molecular entities used in spatial analysis. In some embodiments, some or all of the features in an array are functionalized for analyte capture. Exemplary substrates are described in Section (II)(c) of WO 2020/176788 and/or U.S. Patent Application Publication No. 2020/0277663. Exemplary features and geometric attributes of an array can be found in Sections (II)(d)(i), (II)(d)(iii), and (II)(d)(iv) of WO 2020/176788 and/or U.S. Patent Application Publication No. 2020/0277663.

Generally, analytes and/or intermediate agents (or portions thereof) can be captured when contacting a biological sample with a substrate including capture probes (e.g., a substrate with capture probes embedded, spotted, printed, fabricated on the substrate, or a substrate with features (e.g., beads, wells) comprising capture probes). As used herein, "contact," "contacted," and/or "contacting," a biological sample with a substrate refers to any contact (e.g., direct or indirect) such that capture probes can interact (e.g., bind covalently or non-covalently (e.g., hybridize)) with analytes from the biological sample. Capture can be achieved actively (e.g., using electrophoresis) or passively (e.g., using diffusion). Analyte capture is further described in Section (II)(e) of WO 2020/176788 and/or U.S. Patent Application Publication No. 2020/0277663.

In some cases, spatial analysis can be performed by attaching and/or introducing a molecule (e.g., a peptide, a lipid, or a nucleic acid molecule) having a barcode (e.g., a spatial barcode) to a biological sample (e.g., to a cell in a biological sample). In some embodiments, a plurality of molecules (e.g., a plurality of nucleic acid molecules) having a plurality of barcodes (e.g., a plurality of spatial barcodes) are introduced to a biological sample (e.g., to a plurality of cells in a biological sample) for use in spatial analysis. In some embodiments, after attaching and/or introducing a molecule having a barcode to a biological sample, the biological sample can be physically separated (e.g., dissociated) into single cells or cell groups for analysis. Some such methods of spatial analysis are described in Section (III) of WO 2020/176788 and/or U.S. Patent Application Publication No. 2020/0277663.

In some cases, spatial analysis can be performed by detecting multiple oligonucleotides that hybridize to an analyte. In some instances, for example, spatial analysis can be performed using RNA-templated ligation (RTL). Methods of RTL have been described previously. See, e.g., Credle et al., Nucleic Acids Res. 2017 Aug 21;45(14):e128. Typically, RTL includes hybridization of two oligonucleotides to adjacent sequences on an analyte (e.g., an RNA molecule, such as an mRNA molecule). In some instances, the oligonucleotides are DNA molecules. In some instances, one of the oligonucleotides includes at least two ribonucleic acid bases at the 3' end and/or the other oligonucleotide includes a phosphorylated nucleotide at the 5' end. In some instances, one of the two oligonucleotides includes a capture domain (e.g., a poly(A) sequence, a non-homopolymeric sequence). After hybridization to the analyte, a ligase (e.g., SplintR ligase) ligates the two oligonucleotides together, creating a ligation product. In some instances, the two oligonucleotides hybridize to sequences that are not adjacent to one another. For example, hybridization of the two oligonucleotides creates a gap between the hybridized oligonucleotides. In some instances, a polymerase (e.g., a DNA polymerase) can extend one of the oligonucleotides prior to ligation. After ligation, the ligation product is released from the analyte. In some instances, the ligation product is released using an endonuclease (e.g., RNAse H). The released ligation product can then be captured by capture probes (e.g., instead of direct capture of an analyte) on an array, optionally amplified, and sequenced, thus determining the location and optionally the abundance of the analyte in the biological sample.

During analysis of spatial information, sequence information for a spatial barcode associated with an analyte is obtained, and the sequence information can be used to provide information about the spatial distribution of the analyte in the biological sample. Various methods can be used to obtain the spatial information. In some embodiments, specific capture probes and the analytes they capture are associated with specific locations in an array of features on a substrate. For example, specific spatial barcodes can be associated with specific array locations prior to array fabrication, and the sequences of the spatial barcodes can be stored (e.g., in a database) along with specific array location information, so that each spatial barcode uniquely maps to a particular array location.

Alternatively, specific spatial barcodes can be deposited at predetermined locations in an array of features during fabrication such that at each location, only one type of spatial barcode is present so that spatial barcodes are uniquely associated with a single feature of the array. Where necessary, the arrays can be decoded using any of the methods described herein so that spatial barcodes are uniquely associated with array feature locations, and this mapping can be stored as described above.

When sequence information is obtained for capture probes and/or analytes during analysis of spatial information, the locations of the capture probes and/or analytes can be determined by referring to the stored information that uniquely associates each spatial barcode with an array feature location. In this manner, specific capture probes and captured analytes are associated with specific locations in the array of features. Each array feature location represents a position relative to a coordinate reference point (e.g., an array location, a fiducial marker) for the array. Accordingly, each feature location has an "address" or location in the coordinate space of the array.

Some exemplary spatial analysis workflows are described in the Exemplary Embodiments section of WO 2020/176788 and/or U.S. Patent Application Publication No. 2020/0277663. See, for example, the Exemplary embodiment starting with "In some non-limiting examples of the workflows described herein, the sample can be immersed..." of WO 2020/176788 and/or U.S. Patent Application Publication No. 2020/0277663. See also, e.g., the Visium Spatial Gene Expression Reagent Kits User Guide (e.g., Rev C, dated June 2020), and/or the Visium Spatial Tissue Optimization Reagent Kits User Guide (e.g., Rev C, dated July 2020).

In some embodiments, spatial analysis can be performed using dedicated hardware and/or software, such as any of the systems described in Sections (II)(e)(ii) and/or (V) of WO 2020/176788 and/or U.S. Patent Application Publication No. 2020/0277663, or any of one or more of the devices or methods described in Sections *Control Slide for Imaging*, *Methods of Using Control Slides and Substrates for, Systems of Using Control Slides and Substrates for Imaging,* and/or *Sample and Array Alignment Devices and Methods*, *Informational labels* of WO 2020/123320.

Suitable systems for performing spatial analysis can include components such as a chamber (e.g., a flow cell or sealable, fluid-tight chamber) for containing a biological sample. The biological sample can be mounted for example, in a biological sample holder. One or more fluid chambers can be connected to the chamber and/or the sample holder via fluid conduits, and fluids can be delivered into the chamber and/or sample holder via fluidic pumps, vacuum sources, or other devices coupled to the fluid conduits that create a pressure gradient to drive fluid flow. One or more valves can also be connected to fluid conduits to regulate the flow of reagents from reservoirs to the chamber and/or sample holder.

The systems can optionally include a control unit that includes one or more electronic processors, an input interface, an output interface (such as a display), and a storage unit (e.g., a solid state storage medium such as, but not limited to, a magnetic, optical, or other solid state, persistent, writeable and/or re-writeable storage medium). The control unit can optionally be connected to one or more remote devices via a network. The control unit (and components thereof) can generally perform any of the steps and functions described herein. Where the system is connected to a remote device, the remote device (or devices) can perform any of the steps or features described herein. The systems can optionally include one or more detectors (e.g., CCD, CMOS) used to capture images. The systems can also optionally include one or more light sources (e.g., LED-based, diode-based, lasers) for illuminating a sample, a substrate with features, analytes from a biological sample captured on a substrate, and various control and calibration media.

The systems can optionally include software instructions encoded and/or implemented in one or more of tangible storage media and hardware components such as application specific integrated circuits. The software instructions, when executed by a control unit (and in particular, an electronic processor) or an integrated circuit, can cause the control unit, integrated circuit, or other component executing the software instructions to perform any of the method steps or functions described herein.

In some cases, the systems described herein can detect (e.g., register an image) the biological sample on the array. Exemplary methods to detect the biological sample on an array are described in PCT Application No. 2020/061064 and/or U.S. Patent Application Serial No. 16/951,854.

Prior to transferring analytes from the biological sample to the array of features on the substrate, the biological sample can be aligned with the array. Alignment of a biological sample and an array of features including capture probes can facilitate spatial analysis, which can be used to detect differences in analyte presence and/or level within different positions in the biological sample, for example, to generate a three-dimensional map of the analyte presence and/or level. Exemplary methods to generate a two- and/or three-dimensional map of the analyte presence and/or level are described in PCT Application No. 2020/053655 and spatial analysis methods are generally described in WO 2020/061108 and/or U.S. Patent Application Serial No. 16/951,864.

In some cases, a map of analyte presence and/or level can be aligned to an image of a biological sample using one or more fiducial markers, e.g., objects placed in the field of view of an imaging system which appear in the image produced, as described in the *Substrate Attributes* Section, *Control Slide for Imaging* Section of WO 2020/123320, PCT Application No. 2020/061066, and/or U.S. Patent Application Serial No. 16/951,843. Fiducial markers can be used as a point of reference or measurement scale for alignment (e.g., to align a sample and an array, to align two substrates, to determine a location of a sample or array on a substrate relative to a fiducial marker) and/or for quantitative measurements of sizes and/or distances.

### Spatial Assay for Transposase Accessible Chromatin

The human body includes a large collection of diverse cell types, each providing a specialized and context-specific function. Understanding a cell's chromatin structure can reveal information about the cell's function. Open chromatin, or accessible chromatin, or accessible genomic DNA, is often indicative of transcriptionally active sequences, e.g., genes, in a particular cell. Further understanding the transcriptionally active regions within chromatin will enable identification of which genes contribute to a cell's function and/or phenotype.

Methods have been developed to study epigenomes, e.g., chromatin accessibility assays (ATAC-seq) or identifying proteins associated with chromatin e.g., (ChIP-seq). These assays help identify, for example, regulators (e.g., cis regulators and/or trans regulators) that contribute to dynamic cellular phenotypes. While ATAC-Seq and ChIP-Seq have been invaluable in defining epigenetic variability within a cell population, conventional applications of these methods are limited in their ability to spatially resolve the three dimensional structures and associated genes that promote cellular variation.

Thus, the present disclosure relates generally to the spatial tagging and analysis of nucleic acids. In some embodiments, provided herein are methods that utilize a transposase enzyme to engage and fragment, for example, the accessible (e.g., open chromatin) genomic DNA and enable the simultaneous capture of DNA and RNA from a biological sample, thus revealing epigenomic insights regarding the structural features contributing to cellular regulation.

Provided herein are methods for determining genomic DNA accessibility including (a) a biological sample on an array comprising a plurality of capture probes, where a capture probe of the plurality of capture probes comprises: (i) a spatial barcode and (ii) a capture domain; (b) contacting a plurality of splint oligonucleotides to the biological sample, wherein a splint oligonucleotide binds to the capture domain; (c) contacting a transposome to the biological sample to insert transposon end sequences into accessible genomic DNA, thereby generating fragmented genomic DNA; (d) hybridizing the fragmented genomic DNA to the splint oligonucleotide and ligating the transposon end sequences of the fragmented genomic DNA to the capture probe, thereby generating ligated transposon end sequences; (e) releasing one or more non-ligated transposon end sequences from the ligated transposon end sequences; (f) determining (i) all or a portion of a sequence of the spatial barcode or a complement thereof, and (ii) all or a portion of a sequence of the fragmented genomic DNA, or a complement thereof, and using the determined sequences of (i) and (ii) to determine genomic DNA accessibility in the biological sample.

In some embodiments, steps (d) and (e) are performed sequentially. In some embodiments, steps (d) and (e) are performed simultaneously. For example, some tagmented DNA fragments can be captured with non-ligated transposon end sequences still hybridized. In such examples, the non-ligated transposon end sequences are released after capture of the tagmented DNA. In some embodiments, the non-ligated transposon end sequences are released prior to capture by the capture domain.

Also provided herein are methods for determining genomic DNA accessibility including (a) a biological sample on an array comprising a plurality of capture probes, wherein a capture probe of the plurality of capture probes comprises: (i) a spatial barcode and (ii) a capture domain; (b) contacting a transposome to the biological sample to insert transposon end sequences into accessible genomic DNA, thereby generating fragmented genomic DNA; (c) hybridizing the transposon end sequences of the fragmented genomic DNA to the capture domain of the capture probe; (d) releasing one or more transposon end sequences not bound to the capture domain; (e) determining (i) all or a portion of a sequence of the spatial barcode or a complement thereof, and (ii) all or a portion of a sequence of the fragmented genomic DNA, or a complement thereof, and using the determined sequences of (i) and (ii) to determine genomic DNA accessibility in the biological sample.

In some embodiments, steps (c) and (d) are performed sequentially. In some embodiments, steps (c) and (d) are performed simultaneously. For example, some tagmented DNA fragments can be captured with one or more transposon end sequences still hybridized. In such examples, the one or more transposon end sequences are released after capture of the tagmented DNA. In some embodiments, the one or more transposon end sequences are released prior to capture by the capture domain.

In some embodiments, provided herein are methods for spatial analysis of nucleic acids (e.g., genomic DNA, mRNA) in a biological sample. In some embodiments, an array is provided, wherein the array comprises a plurality of capture probes. In some embodiments, the capture probes may be attached directly to the substrate (e.g., an array comprising a substrate comprising a plurality of capture probes). In some embodiments, the capture probes may be attached indirectly to the substrate. For example, the capture probes can be attached to features on the substrate. In some embodiments, a feature is a bead. In some embodiments, the capture probes comprise a spatial barcode and a capture domain. In some embodiments, the capture probe can be partially double stranded. In some embodiments, the capture probe can bind a complementary oligonucleotide. In some embodiments, the complementary oligonucleotide (e.g., splint oligonucleotide) can have a single stranded portion. In some embodiments, the single stranded portion can hybridize to fragmented (e.g., tagmented) DNA. In some embodiments, a biological sample is treated under conditions sufficient to make nucleic acids in cells of the biological sample (e.g., genomic DNA) accessible to transposon insertion (e.g., tagging the DNA fragments with transposon ends). In some embodiments, a transposon end sequence and a transposase enzyme (collectively, a transposome) are provided to the biological sample such that the transposon end sequence can be inserted into the accessible genomic DNA of cells present in the biological sample. In some embodiments, the transposase enzyme of the transposome complex fragments the genomic DNA and transposon ends are attached to the ends of the genomic DNA fragments (e.g., "tagmenting").

In some embodiments, the biological sample comprising nucleic acids (e.g., genomic DNA, mRNA) is contacted to the substrate such that a capture probe can interact with the fragmented and tagged (e.g., tagmented) genomic DNA. In some embodiments, the biological sample comprising nucleic acids (e.g., genomic DNA, mRNA) is contacted with the substrate such that the capture probe can interact with both the tagmented genomic DNA and the mRNA present in the biological sample (e.g., a first capture probe can bind tagmented genomic DNA, a second capture probe can bind mRNA).

In some embodiments, the location of the capture probe on the substrate can be correlated to a location in the biological sample, thereby spatially determining the location of the tagmented genomic DNA in the biological sample. In some embodiments, the location of the capture probe on the substrate can be correlated to a location in the biological sample, thereby spatially determining the location of the tagmented genomic DNA and mRNA in the biological sample.

### Spatial A TAC-seq

In some embodiments, of any of the spatial analysis methods described herein, ATAC-seq is used to generate genome-wide chromatin accessibility maps. These genome-wide accessibility maps can be integrated with additional genome-wide profiling data (e.g., RNA-seq, ChIP-seq, Methyl-Seq) to produce gene regulatory interaction maps that facilitate understanding of transcriptional regulation. For example, interrogation of genome-wide accessibility maps can reveal the underlying transcription factors and the transcription factor motifs responsible for chromatin accessibility at a given genomic location. Correlating changes in chromatin accessibility with changes in gene expression (RNA-seq), changes in transcription factor binding (e.g., ChIP-seq) and/or changes in DNA methylation levels (e.g., Methyl-seq) can identify the transcription regulation driving these changes. In disease states, there is often an imbalance in transcriptional regulation. Thus, analyzing both chromatin accessibility and, for example, gene expression using spatial analysis methods enables identification of the underlying imbalances in transcriptional regulation, and potentially the causes thereof.

In some embodiments, where spatial determination of the location of analytes includes a concurrent analysis of different types of analytes from a single cell or a subpopulation of cells within a biological sample (e.g., a tissue section), an additional layer of spatial information can be integrated into the genome regulatory interaction maps. In some embodiments, the spatial determining of analytes can be done on whole genomes. In some embodiments, the spatial profiling can be done on an immobilized biological sample.

In some embodiments, the genome-wide chromatin accessibility maps generated by spatial ATAC-seq can be used for cell type identification. For example, traditional cell type classification relies on mRNA expression levels but chromatin accessibility can be more adept at capturing cell identity. Furthermore, in some embodiments, correlations between transcriptionally active regions (e.g., open chromatin, accessible) with expression profiles (e.g., expression profiles of mRNA) can be determined in a spatial manner.

### Permeabilizing the biological sample

The present disclosure generally describes methods of tagmenting genomic DNA to generate DNA fragments in a biological sample. In some examples, a chemical or enzymatic "pre-permeabilization" of biological samples immobilized on a substrate can be employed to allow the DNA in the biological sample to become accessible to a transposase enzyme (e.g. in a transposome complex). In some embodiments, permeabilizing the biological sample can be a two-step process (e.g., pre-permeabilization treatment, followed by a permeabilization treatment). In some embodiments, permeabilizing the biological sample can be a one-step process (e.g., a single permeabilization treatment sufficient to permeabilize the cellular and nuclear membranes in the biological sample).

In some embodiments, pre-permeabilization can include an enzymatic or chemical condition. In some embodiments, pre-permeabilization can be performed with an enzyme (e.g., a protease). In some embodiments, in a non-limiting way, the protease can include trypsin, pepsin, dispase, papain, or collagenase. In some embodiments, pre-permeabilization can include an enzymatic treatment with pepsin. In some embodiments, pre-permeabilization can include pepsin in 0.5M acetic acid. In some embodiments, pre-permeabilization can include pepsin in an Exonuclease-1 buffer. In some embodiments, the pH of the buffer can be acidic. In some embodiments, pre-permeabilization can include enzymatic treatment with collagenase. In some embodiments, pre-permeabilization can include collagenase in HBSS buffer. In some embodiments, the HBSS buffer can include bovine serum albumin (BSA). In some embodiments, pre-permeabilization can last for about 1 to minute to about 20 minutes. In some embodiments, pre-permeabilization can last for about 2, about 3, about 4, about 5, about 6, about 7, about 8, about 9, about 10, about 11, about 12, about 13, about 14, about 15, about 16, about 17, about 18, or about 19 minutes. In some embodiments, pre-permeabilization can last for about 10 minutes to about one hour. For example, in some embodiments, pre-permeabilization can last for about 20, about 30, about 40, or about 50 minutes.

In some embodiments, permeabilizing the biological sample comprises an enzymatic treatment. In some embodiments, the enzymatic treatment can be a pepsin enzyme, or a pepsin-like enzyme treatment. In some embodiments, the enzymatic treatment can be a protease treatment. In some embodiments, enzymatic treatment can be performed in the presence of reagents. In some embodiments, the enzymatic treatment (e.g., pre-permeabilization) can include contacting the biological specimen with an acidic solution including a protease enzyme. In some embodiments, the reagent can be HCl. In some embodiments, the reagent can be acetic acid. In some embodiments, the concentration of HCl can be about 100mM. In some embodiments, the about 100mM HCl can have a pH of around, or about 1.0. In some embodiments, an additional reagent can be 0.5M acetic acid, having a pH of around, or about 2.5. It is noted that enzymatic treatment of the biological sample can have different effects on tagmentation. For example, enzymatic treatment with pepsin and 100mM HCl can result in tagmentation of chromatin regardless of chromatin accessibility. In some embodiments, enzymatic treatment with pepsin and 0.5M acetic acid can result in tagmentation of chromatin that can retain a nucleosomal pattern indicative of chromatin accessibility.

In some embodiments, the enzymatic treatment can comprise contacting the biological sample with a reaction mixture (e.g., solution) comprising an aspartyl protease (e.g., pepsin) in an acidic buffer, e.g., a buffer with a pH of about 4.0 or less, such as about 3.0 or less, e.g., about 0.5 to about 3.0, or about 1.0 to about 2.5. In some embodiments, the aspartyl protease is a pepsin enzyme, pepsin-like enzyme, or a functional equivalent thereof. Thus, any enzyme or combination of enzymes in the enzyme commission number 3.4.23.1.

In some embodiments, the enzymatic treatment (e.g., pre-permeabilization) can be performed using collagenase. In some embodiments, enzymatic treatment with collagenase can provide access to the genomic DNA for the transposase while preserving nuclear integrity. In some embodiments, pre-permeabilization (e.g., enzymatic treatment) with collagenase yields nucleosomal patterns generally associated with chromatin accessibility. Collagenases can be isolated from *Clostridium histolyticum.* In some embodiments, enzymatic treatment with a zinc endopeptidase (e.g., collagenase) with reagents and under conditions suitable for proteolytic activity comprises a buffered solution with a pH of about 7.0 to about 8.0 (e.g., about 7.4). Collagenases are zinc endopeptidases and can be inhibited by either EDTA or EGTA, or both. Therefore, in some embodiments, the biological sample can be contacted with a zinc endopeptidase (e.g., collagenase) in the absence of a chelator of divalent cations, (e.g., EDTA, EGTA). In some embodiments, it can be useful to stop the zinc endopeptidase (e.g., collagenase) and the permeabilization step can be stopped (e.g., inhibited) by contacting the biological sample with a chelator of divalent cations (e.g., EDTA, EGTA).

In some embodiments, the zinc endopeptidase is a collagenase enzyme, collagenase-like enzyme, or a functional equivalent thereof. In such embodiments, any enzyme or combination of enzymes in the enzyme commission number 3.4.23.3 can be used in accordance with materials and methods described herein. In some embodiments, the collagenase is one or more collagenases from the following group, (UniProtKB/Swiss-Prot accession numbers): P43153/COLA_CLOPE; P43154/COLA_VIBAL; Q9KRJ0/COLA VIBCH; Q56696/COLA VIBPA; Q8D4Y9/COLA VIBVU; Q9X721/COLG HATHI; Q46085/COLH_HATHI; Q899Y1/COLT_CLOTE URSTH and functional variants and derivatives thereof (described herein), or a combination thereof.

Methods of permeabilizing biological samples are well known in the art. It will be known to a person skilled in the art that different sources of biological samples can be treated with different reagents (e.g., proteases, RNAses, detergents, buffers) and under different conditions (e.g., pressure, temperature, concentration, pH, time). In some embodiments, permeabilizing the biological sample can comprise reagents and conditions to sufficiently disrupt the cell membrane of the biological sample to capture nucleic acids (e.g., mRNA). In some embodiments, permeabilizing the biological sample can comprise reagents and conditions to sufficiently disrupt the nuclear membrane of the biological sample to capture nucleic acids (e.g., genomic DNA). In some embodiments, commercially available proteases isolated from their native (e.g., animal, microbial source) can be used. In some embodiments, proteases produced recombinantly (e.g., bacterial expression system, viral expression system) can be used. In some embodiments, pre-permeabilizing and permeabilizing a biological sample can be a one-step process (e.g., enzymatic treatment). In some embodiments, pre-permeabilizing and permeabilizing a biological sample can be a two-step process (e.g., enzymatic treatment, followed by chemical or detergent treatment).

In some embodiments, the chemical permeabilization conditions comprise contacting the biological specimen with an alkaline solution, e.g. a buffered solution with a pH of about 8.0 to about 11.0, such as about 8.5 to about 10.5 or about 9.0 to about 10.0, e.g. about 9.5. In some embodiments, the buffer is a glycine-KOH buffer. Other buffers are known in the art.

In some embodiments, a biological sample can be treated with a detergent following an enzymatic treatment (e.g., permeabilization following a pre-permeabilization step). Detergents are known in the art. Any suitable detergent can be used, including, in a non-limiting way NP-40 or equivalent, Digitonin, Tween-20, IGEPAL-40 or equivalent, Saponin, SDS, Pitsop2, Triton X-100 or combinations thereof. In some embodiments, a biological sample can be treated with other chemicals known to permeabilize cellular membranes. As further exemplified in the examples below, detergents described herein can be used at a concentration of between about 0.001% (v/v) to about 5% (v/v). In some embodiments, detergents described herein can be used at a concentration of about 0.01% (v/v), about 0.02% (v/v), about 0.03% (v/v), about 0.04% (v/v), about 0.05% (v/v), about 0.06% (v/v), about 0.07% (v/v), about 0.08%, or about 0.09%. In some embodiments, detergents described herein can be used at a concentration of about 0.1% (v/v), about 0.2% (v/v), about 0.3% (v/v), about 0.4% (v/v), about 0.5% (v/v), about 0.6% (v/v), about 0.7% (v/v), about 0.8% (v/v), about 0.9% (v/v), about 1.0% (v/v), or about 1.1% to about 10% (v/v) or more. In some embodiments, detergents described herein can be used at a concentration of about 2% (v/v), about 3% (v/v), about 4% (v/v), about 5% (v/v), about 6% (v/v), about 7% (v/v), about 8% (v/v), or about 9% (v/v), or about 10% (v/v).

Additional methods for sample permeabilization are described, for example, in Jamur et al., Method Mol. Biol. 588:63-66, 2010, the entire contents of which are incorporated herein by reference. Any suitable method for biological sample permeabilization can generally be used in connection with the biological samples described herein.

Different sources of biological samples can be treated with different reagents (e.g., proteases, RNAses, detergents, buffers) and under different suitable conditions (e.g., pressure, temperature, concentration, pH, time) to achieve sufficient pre-permeabilization and permeabilization to capture nucleic acids (e.g., genomic DNA, mRNA).

In some embodiments, the reaction mixture (e.g., solution) including the proteases described herein can contain other reagents, (e.g., buffer, salt, etc.) sufficient to ensure that the proteases are functional. For instance, the reaction mixture can further include an albumin protein, (e.g., BSA). In some embodiments, the reaction mixture (e.g., solution) including the collagenase enzyme (or functional variant or derivative thereof) includes an albumin protein, (e.g., BSA).

In some embodiments, there is one or more wash steps between pre-permeabilization and permeabilization of a biological sample. For example, it may be preferential to wash as much of the pre-permeabilization solution off of the biological sample prior to adding a permeabilization solution. Therefore, in some embodiments the biological sample is washed, for example with a SSC wash solution, after pre-permeabilization to remove the pre-permeabilization reagents and before applying permeabilization reagents to the biological sample. In some embodiments, the permeabilization solution is removed from the biological sample prior to the addition of the transposome reagents for tagmentation of the released genomic DNA. One or more washes may also be performed post permeabilization and pre-tagmentation, for example using a SSC solution. In some embodiments, there are no wash steps between permeabilization of the biological sample and tagmentation of the genomic DNA.

### Tagmentation

Transposase enzymes and transposons can be utilized in methods of spatial genomic analysis. Generally, transposition is the process by which a specific genetic sequence (e.g., a transposon sequence) is relocated from one place in a genome to another. Many transposition methods and transposable elements are known in the art (e.g., DNA transposons, retrotransposons, autonomous transposons, non-autonomous transposons). One non-limiting example of a transposition event is conservative transposition. Conservative transposition is a non-replicative mode of transposition in which the transposon is completely removed from the genome and reintegrated into a new locus, such that the transposon sequence is conserved, (e.g., a conservative transposition event can be thought of as a "cut and paste" event) (*See*, e.g., Griffiths A. J., et. al., Mechanism of transposition in prokaryotes. An Introduction to Genetic Analysis (7th Ed.). New York: W. H. Freeman (2000)).

In one example, cut and paste transposition can occur when a transposase enzyme binds a sequence flanking the ends of the transposome (e.g., a recognition sequence, e.g., a mosaic end sequence, a transposon sequence). A transposome (e.g., a transposition complex) forms and the endogenous DNA can be manipulated into a pre-excision complex such that two transposase enzymes can interact. In some embodiments, when the transposases interact double stranded breaks are introduced into the DNA. The transposase enzymes can locate and bind a target site in the DNA, create a double stranded break, and insert the transposon end sequence (*See,* e.g., Skipper, K.A., et. al., DNA transposon-based gene vehicles-scenes from an evolutionary drive, J Biomed Sci., 20: 92 (2013) doi:10.1186/1423-0127-20-92). Alternative cut and paste transposases include Tn552 (College, et al, J. BacterioL, 183: 2384-8, 2001; Kirby C et al, Mol. Microbiol, 43: 173-86, 2002), Tyl (Devine & Boeke, Nucleic Acids Res., 22: 3765-72, 1994 and International Publication WO 95/23875), Tn7 (Craig, N L, Science. 271 : 1512, 1996; Craig, N L, Review in: Curr Top Microbiol Immunol, 204:27-48, 1996), Tn/O and IS10 (Kleckner N, et al, Curr Top Microbiol Immunol, 204:49-82, 1996), Mariner transposase (Lampe D J, et al, EMBO J., 15: 5470-9, 1996), Tel (Plasterk R H, Curr. Topics Microbiol. Immunol, 204: 125-43, 1996), P Element (Gloor, G B, Methods Mol. Biol, 260: 97- 114, 2004), Tn3 (Ichikawa & Ohtsubo, J Biol. Chem. 265: 18829-32, 1990), bacterial insertion sequences (Ohtsubo & Sekine, Curr. Top. Microbiol. Immunol. 204: 1-26, 1996), retroviruses (Brown, et al, Proc Natl Acad Sci USA, 86:2525-9, 1989), and retrotransposon of yeast (Boeke & Corces, Annu Rev Microbiol. 43:403-34, 1989). More examples include IS5, TnlO, Tn903, IS911, and engineered versions of transposase family enzymes (Zhang et al, (2009) PLoS Genet. 5:el000689. Epub 2009 Oct. 16; Wilson C. et al (2007) J. Microbiol. Methods 71:332-5).

Transposome-mediated fragmentation and tagging ("tagmentation") is a process of transposase-mediated fragmentation and tagging of DNA. A transposome is a complex of a transposase enzyme and DNA which comprises a transposon end sequence (also known as "transposase recognition sequence" or "mosaic end" (MEs)). In some methods of spatial genomic analysis, DNA is fragmented in such a manner that a functional sequence such as a sequence complementary to a capture domain of a capture probe (e.g., capture domain of a splint oligonucleotide) is inserted into the fragmented DNA (e.g., the fragmented DNA is "tagged"), such that the sequence (e.g. an adapter, e.g., Nextera sequence) can hybridize to the capture probe. In some embodiments, the capture probe is present on a substrate. In some embodiments, the capture probe (e.g., a capture probe and a splint oligonucleotide) is present on a feature. A transposase dimer (e.g., in the case of Tn5 transposase system) in conjunction with a transposon sequence (e.g., transposome) is able to simultaneously fragment DNA based on its transposon recognition sequences and ligate DNA from the transposome (e.g., transposon sequence) to the fragmented DNA (e.g., tagmented DNA). This system has been adapted using hyperactive transposase enzymes and modified DNA molecules (adaptors) comprising MEs to fragment DNA and tag both strands of DNA duplex fragments with functional DNA molecules (e.g., primer binding sites). For instance, the Tn5 transposase may be produced as purified protein monomers. Tn5 transposase is also commercially available (e.g., manufacturer Illumina, Illumina.com, Catalog No. 15027865, TD Tagment DNA Buffer Catalog No. 15027866). These can be subsequently loaded with the oligonucleotides of interest, e.g., ssDNA oligonucleotides containing MEs (e.g., transposon sequences) for Tn5 recognition and additional functional sequences (e.g., Nextera adapters, e.g., primer binding sites) are annealed to form a dsDNA mosaic end oligonucleotide (MEDS) that is recognized by Tn5 during dimer assembly (e.g., transposome dimerization). In some embodiments, a hyperactive Tn5 transposase can be loaded with adapters (e.g., oligonucleotides of interest) which can simultaneously fragment and tag a genome.

As used herein, the term "tagmentation" refers to a step in the Assay for Transposase Accessible Chromatin using sequencing (ATAC-seq). (*See,* e.g., Buenrostro, J. D., Giresi, P. G., Zaba, L. C, Chang, H. Y., Greenleaf, W. J., Transposition of native chromatin for fast and sensitive epi genomic profiling of open chromatin, DNA-binding proteins and nucleosome position, Nature Methods, 10 (12): 1213-1218 (2013)). ATAC-seq identifies regions of open chromatin using a hyperactive prokaryotic Tn5-transposase, which preferentially inserts into accessible chromatin and tags the sites with adaptors (Buenrostro, J.D., et. al., Transposition of native chromatin for fast and sensitive epigenomic profiling of open chromatin, DNA-binding proteins and nucleosome position. Nat Methods, 10: 1213-1218 (2013)).

As used herein "accessible chromatin" or "open chromatin" or "accessible genomic DNA" refers to portions of a genome that are nucleosome-depleted regions that can be bound by proteins and play various roles in nuclear organization, gene transcription, and are generally considered transcriptionally active regions of DNA (Zhang, Q., et al., Genome-wide open chromatin regions and their effects on the regulation of silk protein genes in Bombyx mori, Scientific Reports, 7: 12919 (2017).

In some embodiments, the step of fragmenting the genomic DNA in cells of the biological sample comprises contacting the biological sample containing the genomic DNA with the transposase enzyme (e.g., a transposome, e.g., a reaction mixture (e.g., solution)) including a transposase), under any suitable conditions. In some embodiments, such suitable conditions result in the tagmentation of the genomic DNA of cells present in the biological sample. Typical conditions will depend on the transposase enzyme used and can be determined using routine methods known in the art. Therefore, suitable conditions can be conditions (e.g., buffer, salt, concentration, pH, temperature, time conditions) under which the transposase enzyme is functional, e.g., in which the transposase enzyme displays transposase activity, particularly tagmentation activity, in the biological sample.

The term "functional", as used herein in reference to transposase enzymes, is meant to include embodiments in which the transposase enzyme can show some reduced activity relative to the activity of the transposase enzyme in conditions that are optimum for the enzyme, e.g., in the buffer, salt and temperature conditions recommended by the manufacturer. Thus, the transposase can be considered to be "functional" if it has at least about 50%, e.g., at least about 60%, about 70%, about 80%, about 85%, about 90%, about 95%, about 96%, about 97%, about 98%, about 99%, or about 100%, activity relative to the activity of the transposase in conditions that are optimum for the transposase enzyme.

In one non-limiting example, the reaction mixture comprises a transposome in a buffered solution (e.g., Tris-acetate) having a pH of about 6.5 to about 8.5, e.g., about 7.0 to about 8.0 such as about 7.5. Additionally or alternatively, the reaction mixture can be used at any suitable temperature, such as about 10°C to about 55°C, e.g., about 10°C to about 54°C, about 11°C to about 53°C, about 12°C to about 52°C, about 13°C to about 51°C, about 14°C to about 50°C, about 15°C to about 49°C, about 16°C to about 48°C, about 17°C to about 47°C, e.g., about 10°C, about 12°C, about 15°C, about 18°C, about 20°C, about 22°C, about 25°C, about 28°C, about 30°C, about 33°C, about 35°C, or about 37°C, preferably about 30°C to about 40°C, e.g., about 37°C. In some embodiments, the transposome can be contacted with the biological sample for about 10 minutes to about one hour. In some embodiments, the transposome can be contacted with the biological sample for about 20, about 30, about 40, or about 50 minutes. In some embodiments, the transposome can be contacted with the biological sample for about 1 hour to about 4 hours.

In some embodiments, the transposase enzyme of the transposome complex is a Tn5 transposase, or a functional derivate or variant thereof. (See, e.g., Reznikoff et al, WO 2001/009363, U.S. Patent Nos. 5,925,545, 5,965,443, 7,083,980, and 7,608,434, and Goryshin and Reznikoff, J. Biol. Chem. 273:7367, (1998), which are herein incorporated by reference). In some embodiments, the Tn5 transposase is a hyper Tn5 transposase, or a functional derivate or variate thereof (US patent 9,790,476, incorporated herein by reference). For example, the Tn5 transposase can be a fusion protein (e.g., a Tn5 fusion protein). Tn5 is a member of the RNase superfamily of proteins. The Tn5 transposon is a composite transposon in which two near-identical insertion sequences (IS50L and IS50R) flank three antibiotic resistance genes. Each IS50 contains two inverted 19-bp end sequences (ESs), an outside end (OE) and an inside end (IE). Wild-type Tn5 transposase enzyme is generally inactive (e.g., low transposition event activity). However, amino acid substitutions can result in hyperactive variants or derivatives. In one non-limiting example, amino acid substitution, L372P, substitutes a leucine amino acid for a proline amino acid which results in an alpha helix break, thus inducing a conformational change to the C-terminal domain. The alpha helix break separates the C-terminal domain and N-terminal domain sufficiently to promote higher transposition event activity (See, Reznikoff, W.S., Tn5 as a model for understanding DNA transposition, Mol Microbiol, 47(5): 1199-1206 (2003)). Other amino acid substitutions resulting in hyperactive Tn5 are known in the art. For example, the improved avidity of the modified transposase enzyme (e.g., modified Tn5 transposase enzyme) for the repeat sequences for OE termini (class (1) mutation) can be achieved by providing a lysine residue at amino acid 54, which is glutamic acid in wild-type Tn5 transposase enzyme (See U.S. Patent No. 5.925,545). The mutation strongly alters the preference of the modified transposase enzyme (e.g., modified Tn5 transposase enzyme) for OE termini, as opposed to IE termini. The higher binding of this mutation, known as EK54, to OE termini results in a transposition rate that is about 10-fold higher than is seen with wild-type transposase enzyme (e.g., wild type Tn5 transposase enzyme). A similar change at position 54 to valine (e.g., EV54) also results in somewhat increased binding/transposition for OE termini, as does a threonine to proline change at position 47 (e.g., TP47; about 10-fold higher) (See U.S. Patent No. 5.925,545).

Other examples of modified transposase enzymes (e.g., modified Tn5 transposase enzymes) are known. For example, a modified Tn5 transposase enzyme that differs from wild-type Tn5 transposase enzyme in that it binds to the repeat sequences of the donor DNA with greater avidity than wild-type Tn5 transposase enzyme and also is less likely than the wild-type transposase enzyme to assume an inactive multimeric form (U.S. Patent No. 5,925,545, which is incorporated by reference in its entirety). Furthermore, techniques generally describing introducing any transposable element (e.g., Tn5) from a donor DNA (e.g., adapter sequence, e.g., Nextera adapters (e.g., top and bottom adapter) into a target are known in the art. (See, e.g., U.S. Patent No. 5,925,545). Further study has identified classes of mutations resulting in a modified transposase enzyme (e.g., modified Tn5 transposase enzyme) (See, U.S. Patent No. 5,965,443, which is incorporated by reference in its entirety). For example, a modified transposase enzyme (e.g., modified Tn5 transposase enzyme) with a "class 1 mutation" binds to repeat sequences of donor DNA with greater avidity than wild-type Tn5 transposase enzyme. Additionally, a modified transposase enzyme (e.g., modified Tn5 transposase enzyme) with a "class 2 mutation" is less likely than the wild-type Tn5 transposase enzyme to assume an inactive multimeric form. It has been shown that a modified transposase enzyme that contains both a class 1 and a class 2 mutation can induce at least about 100-fold (+10%) more transposition than the wild-type transposase enzyme, when tested in combination with an in vivo conjugation assay as described by Weinreich, M.D., "Evidence that the cis Preference of the Tn5 Transposase is Caused by Nonproductive Multimerization," Genes and Development 8:2363-2374 (1994), incorporated herein by reference (See e.g., U.S. Patent No. 5,965,443). Further, under sufficient conditions, transposition using the modified transposase enzyme (e.g., modified Tn5 transposase enzyme) may be higher. A modified transposase enzyme containing only a class 1 mutation can bind to the repeat sequences with sufficiently greater avidity than the wild-type Tn5 transposase enzyme such that a Tn5 transposase enzyme induces about 5- to about 50-fold more transposition than the wild-type transposase enzyme, when measured in vivo. A modified transposase enzyme containing only a class 2 mutation (e.g., a mutation that reduces the Tn5 transposase enzyme from assuming an inactive form) is sufficiently less likely than the wild-type Tn5 transposase enzyme to assume the multimeric form that such a Tn5 transposase enzyme also induces about 5-to about 50-fold more transposition than the wild- type transposase enzyme, when measured in vivo (See U.S. Patent No. 5,965,443)

Other methods of using a modified transposase enzyme (e.g., modified Tn5 transposase enzyme) are further generally described in U.S. Patent No. 5,965,443 and US Patent No. 9,790,476. For example, a modified transposase enzyme could provide selective markers to target DNA, to provide portable regions of homology to a target DNA, to facilitate insertion of specialized DNA sequences into target DNA, to provide primer binding sites or tags for DNA sequencing, or to facilitate production of genetic fusions for gene expression. Studies and protein domain mapping, as well as, to bring together other desired combinations of DNA sequences (combinatorial genetics) (U.S. Patent No. 5,965,443).

Still other methods of inserting a transposable element (e.g., transposon) at random or semi-random locations in chromosomal or extra-chromosomal nucleic acid are known. For example, methods including a step of combining in a biological sample nucleic acid (e.g., genomic DNA) with a synaptic complex that comprises a Tn5 transposase enzyme complexed with a sequence comprising a pair of nucleotide sequences adapted for operably interacting with Tn5 transposase enzyme and a transposable element (e.g., transposon) under conditions that mediate transposition events into the genomic DNA. In this method, a synaptic complex can be formed in vitro under conditions that disfavor or prevent synaptic complexes from undergoing a transposition event. The frequency of transposition (e.g., transposition events) can be increased by using either a hyperactive transposase enzyme (e.g., a mutant transposase enzyme) or a transposable element (e.g., transposon) that contains sequences well adapted for efficient transposition events in the presence of a hyperactive transposase enzyme (e.g., hyperactive Tn5 transposase enzyme), or both (U.S. Patent No. 6,159,736, which is incorporated herein by reference).

Methods, compositions, and kits for treating nucleic acid, and in particular, methods and compositions for fragmenting and tagging DNA using transposon compositions are described in detail in U.S. Patent Application Publication No. US 2010/0120098, U.S. Patent Application Publication No. US2011/0287435, and Satpathy, A.T., et al., Massively parallel single-cell chromatin landscapes of human immune cell development and intratumoral T-cell exhaustion, Nat Biotechnol., 37, 925-936 (2019), the contents of which are herein incorporated by reference in their entireties.

Any transposase enzyme with tagmentation activity, e.g., any transposase enzyme capable of fragmenting DNA and inserting oligonucleotides (e.g., adapters, e.g. Nextera index adapters) to the ends of the fragmented (e.g., tagmented) DNA, can be used. In some embodiments, the transposase is any transposase capable of conservative transposition. In some embodiments, the transposase is a cut and paste transposase. Other kinds of transposases are known in the art and are within the scope of this disclosure. For example, suitable transposase enzymes include, without limitation, Mos-1, HyperMu^{™}, Ts-Tn5, Ts-Tn5059, Hermes, Tn7, a Vibrio species transposase (See e.g., U.S. Patent Application No. 20120301925A1 and WO 2015/069374, the contents of which are herein incorporated by reference in their entireties), or any functional variant or derivative of the previously listed transposase enzymes.

In some embodiments, a hyperactive variant of the Tn5 transposase enzyme is capable of mediating the fragmentation of double-stranded DNA and ligation of synthetic oligonucleotides (e.g., Nextera adapters) at both 5' ends of the DNA in a reaction that takes a short period of time (e.g., about 5 minutes). However, as wild-type end sequences have a relatively low activity, they are sometimes replaced in vitro by hyperactive mosaic end (ME) sequences. A complex of the Tn5 transposase with 19-bp ME facilitates transposition, provided that the intervening DNA is long enough to bring two of these sequences close together to form an active Tn5 transposase enzyme homodimer.

In some embodiments, the Tn5 transposase enzyme, or functional variant or derivative thereof, comprises an amino acid sequence having at least 80% sequence identity to SEQ ID NO: 1. In some embodiments, the Tn5 transposase enzyme, or functional variant or derivative thereof, comprises an amino acid sequence having a sequence identity of at least about 90%, 91%, 92%, 93%, 94%, 95%, 96%, 97%, 98%, 99%, or 100% amino acid sequence identity to SEQ ID NO: 1. In some embodiments, the transposase enzyme is complexed with an adapter including a transposon end sequence. In some embodiments, the Tn5 transposon end sequence comprises a sequence having at least 80% sequence identity to SEQ ID NO: 2. In some embodiments, the Tn5 transposon end sequence comprises an amino acid sequence having a sequence identity of at least about 90%, 91%, 92%, 93%, 94%, 95%, 96%, 97%, 98%, 99%, or 100% amino acid sequence identity to SEQ ID NO. 2.

In some embodiments, the transposase enzyme is a Mu transposase enzyme, or a functional variant or derivative thereof. In some embodiments, the Mu transposase enzyme, or functional variant or derivative thereof, comprises an amino acid sequence having at least 80% identity to SEQ ID NO: 3. In some embodiments, the Mu transposase enzyme, or functional variant or derivative thereof, comprises an amino acid sequence having a sequence identity of at least about 90%, 91%, 92%, 93%, 94%, 95%, 96%, 97%, 98%, 99%, or 100% amino acid sequence identity to SEQ ID NO: 3. In some embodiments, the Mu transposon end sequence (e.g., a transposase recognition sequence) comprises a sequence having at least 80% sequence identity to any one of SEQ ID NOs: 4-9. In some embodiments, the Mu transposon end sequence (e.g., a Mu transposase recognition sequence) comprises a sequence having at least about 90%, 91%, 92%, 93%, 94%, 95%, 96%, 97%, 98%, 99%, or 100% amino acid sequence identity to any one of SEQ ID NOs: 4-9.

In some embodiments, the transposase enzyme is an ISR family transposase, or a functional variant or derivative thereof. For example, the ISR family transposase can be an ISR family transposase described in NCBI Reference Sequence: WP_012128611.1 and/or U.S. Patent No. 9,005,935, which is incorporated herein by reference in its entirety. In some embodiments, the ISR family transposase, or functional variant or derivative thereof, comprises an amino acid sequence having at least 80% identity to SEQ ID NO: 10. In some embodiments, the ISR family transposase, or functional variant or derivative thereof, comprises an amino acid sequence having a sequence identity of at least about 90%, 91%, 92%, 93%, 94%, 95%, 96%, 97%, 98%, 99%, or 100% amino acid sequence identity to SEQ ID NO: 10. In some embodiments, the ISR family transposase transposon end sequence (e.g., transposase recognition sequence) comprises a sequence having at least 80% identity to any one of SEQ ID NOs: 11-13. In some embodiments, the ISR family transposase transposon end sequence (e.g., transposase recognition sequence) comprises a sequence having at least about 90%, 91%, 92%, 93%, 94%, 95%, 96%, 97%, 98%, 99%, or 100% amino acid sequence identity to SEQ ID NOs: 11-13.

The adaptors (e.g., Nextera adaptors) in the complex with the transposase enzyme (e.g., that form part of the transposome, e.g., MEDS described herein) can include partially double stranded oligonucleotides. In some embodiments, there is a first adapter and a second adapter. In some embodiments, the first adapter can be complexed with a first monomer. In some embodiments, the second adapter can be complexed with a second monomer. In some embodiments, the first monomer complexed with the first adapter and the second monomer complexed with the second monomer can be assembled to form a dimer. In some embodiments, the double stranded portion of the adaptors contains transposon end sequences (e.g., Mosaic End (ME)) sequences. In some embodiments, the single stranded portion of the adaptors (e.g., Nextera index adapters) (5' overhang) contains the functional domain or sequence to be incorporated in the tagmented DNA. In some embodiments, the adapters can be Nextera adapters (e.g., index adapter) (for example, reagents including, Nextera DNA Library Prep Kit for ATAC-seq (no longer available), TDE-1 Tagment DNA Enzyme (Catalog No. 15027865), TD Tagment DNA Buffer (Catalog No. 15027866), available from Illumina, Illumina.com). In some embodiments, the sequence incorporated into the tagmented DNA is a sequence complementary to a capture domain of a capture probe. In some embodiments, the sequence complementary to the capture domain of the capture probe is a transposon end sequence. In such embodiments, the functional domain is on the strand of the adaptor that will be ligated to the capture probe. In other words, the functional domain can be located upstream (e.g., 5' to) the ME sequence, e.g., in the 5' overhang of the adapter.

The adaptors (e.g., Nextera index adapters, e.g., first and second adapters) ligated to the tagmented DNA can be any suitable sequence. For example, the sequence can be a viral sequence. In some embodiments, the sequence can be a CRISPR sequence. In some embodiments, the adaptor (e.g., oligonucleotides) ligated to the tagmented DNA can be a CRISPR guide sequence. In some embodiments, the CRISPR guide sequence can target a sequence of interest (e.g., genomic locus of interest e.g., gene specific).

In some embodiments, the ME sequence is a Tn5 transposase recognition sequence. In some embodiments, the mosaic end (e.g., ME) sequence is a Mu transposase recognition sequence. In some embodiments, the ME sequence is a *Vibrio* species transposase recognition sequence.

In some embodiments, a composition comprising a transposase enzyme (e.g., any transposase enzyme described herein) complexed with adapters (e.g., first and second adapters complexed with first and second monomers, respectively) comprising transposon end sequences (e.g., mosaic end sequences) is used in a method for spatially tagging nucleic acids in a biological sample. In some embodiments, a composition comprising a transposase enzyme further comprises a domain that binds to a capture probe as described herein (e.g., Nextera adapter, e.g., first adapter) and a second adapter is used in a method for spatially tagging nucleic acids of a biological sample, such as any of the methods described herein.

In some embodiments, the transposase enzyme can be in the form of a transposome comprising adaptors (MEDS) in which the 5' overhang can be phosphorylated. In some embodiments, the adaptors (e.g., Nextera adaptors, e.g., first and second adapters) may be phosphorylated prior to their assembly with the transposase enzyme to form the transposome. In some embodiments, phosphorylation of adaptors can occur when complexed with a transposase enzyme (e.g., phosphorylation in situ in the transposome).

In some embodiments, the 5' overhang of the adaptor is not phosphorylated prior to its assembly in the transposome. In such embodiments, the 5' overhang can have accessible 5' hydroxyl groups outside of the mosaic-end transposase sequence. In some embodiments, phosphorylation of the 5' overhang of the assembled transposome complexes can be achieved by exposing these 5' ends of transposome complexes to a polynucleotide kinase (e.g., T4-polynucleotide kinase (T4-PNK)) in the presence of ATP.

In some embodiments, tagmenting genomic DNA of the biological sample with a transposome (e.g., any of the transposomes described herein) can comprise a further step of phosphorylating the 5' ends of the adaptors (e.g., the 5' overhangs of the Nextera adaptors, e.g., MEDS) in the transposome complex.

In some embodiments, methods provided herein comprise a step of providing a transposome that has been treated to phosphorylate the 5' ends of the adaptors (e.g., the 5' overhangs of the Nextera adaptors (e.g., first and second adapters), e.g., MEDS) in the transposome complex, thus fragmenting the biological sample with a transposome that has been treated to phosphorylate the 5' ends of the adaptors in the transposome complex.

Any suitable enzyme and/or conditions can be used to phosphorylate the 5' ends of the adaptors (e.g., the 5' overhangs of the adaptors, e.g., MEDS) in the transposome complex, e.g., T4-PNK or T7-PNK. In some embodiments, the phosphorylation reaction can be carried out by contacting the transposome with a polynucleotide kinase (e.g., T4-PNK or T7-PNK) in a buffered solution (e.g., Tris-HCl, pH about 7.0 to about 8.0, e.g., about 7.6) at about 20 to about 40°C, e.g., about 25 to about 37°C, for about 1 to about 60 minutes, e.g., about 5 to about 50, about 10 to about 40, about 20 to about 30 minutes.

In some embodiments, gap filling and ligating breaks can be performed on the fragmented (e.g., tagmented) DNA. For example, the Tn5 transposition event results in a 9 base pair gap between an inserted transposon end sequence and the genomic DNA. In some embodiments, the gap filling is performed between the inserted transposon end sequence and fragmented genomic DNA followed by ligation of the filled gap sequence to the genomic DNA.

In some embodiments, the transposon end sequences adjacent to the 9 base pair gap followed by the fragmented genomic DNA are released. In some examples, the transposon end sequences adjacent to the gap are released (e.g., removed) from the fragmented genomic DNA (e.g., released from the complementary transposon end sequence). In some embodiments, the released transposon end sequences are not ligated to the splint oligonucleotide (e.g., non-ligated transposon end sequences). In some embodiments, the non-ligated transposon end sequences are released with a heat gradient. In some embodiments, the ligated transposon end sequences are ligated to the capture probe. In some embodiments the splint oligonucleotide is hybridized to the capture domain of the capture probe, or a portion thereof, and the remaining transposon end sequence (e.g., ligated transposon end sequence). In some embodiments, a gap filling reaction is performed. In some embodiments, gap filling occurs between the splint oligonucleotide and the fragmented genomic DNA. For example, a gap filling polymerase facilitates nucleic acid extension between the splint oligonucleotide and the fragmented end of the genomic DNA thereby "filling the gap: between the splint oligonucleotide and the fragmented genomic DNA (e.g., a portion of which included the released transposon end sequence).

In some embodiments, the non-ligated transposon end sequences (e.g., transposon end sequences adjacent to 9 base pair gap) are released with a heat gradient from about 20°C to about 90°C, from about 25°C to about 85°C, from about 30°C to about 80°C, from about 35°C to about 75°C, from about 40°C to about 75°C, from about 45°C to about 75°C, 50°C to about 75°C, or about 50°C to about 70°C. In some embodiments, releasing the non-ligated transposon end sequences occurs at about 25°C, about 26°C, about 27°C, about 28°C, about 29°C, about 30°C, about 31°C, about 32°C, about 33°C, about 34°C, about 35°C, about 36°C, about 37°C, about 38°C, about 39°C, about 40°C, about 41°C, about 42°C, about 43°C, about 44°C, about 45°C, about 46°C, about 47°C, about 48°C, about 49°C, about 50°C, about 51°C, about 52°C, about 53°C, about 54°C, about 55°C, about 56°C, about 57°C, about 58°C, about 59°C, about 60°C, about 70°C, about 71°C, about 72°C, about 73°C, about 74°C, about 75°C, about 76°C, about 77°C, about 78°C, about 79°C, about 80°C, about 81°C, about 82°C, about 83°C, about 84°C, about 85°C, about 86°C, about 87°C, about 88°C, about 89°C, or about 90°C.

In some embodiments, releasing the non-ligated transposon end sequences (e.g., transposon end sequences adjacent to the 9 base pair gap) are released with a heat gradient for about 10 minutes to about 150 minutes, from about 20 minutes to about 140 minutes, from about 30 minutes to about 130 minutes, from about 40 minutes to about 120 minutes, from about 40 minutes to about 110 minutes, from about 50 minutes to about 110 minutes, from about 60 minutes to about 100 minutes, from about 70 minutes to about 90 minutes, from about 10 minutes, about 15 minutes, about 20 minutes, about 25 minutes, about 30 minutes, about 35 minutes, about 40 minutes, about 45 minutes, about 50 minutes, about 55 minutes, about 60 minutes, about 65 minutes, about 70 minutes, about 75 minutes, about 80 minutes, about 85 minutes, about 90 minutes, about 95 minutes, about 100 minutes, about 105 minutes, about 110 minutes, about 115 minutes, about 120 minutes, about 125 minutes, about 130 minutes, about 135 minutes, about 140 minutes, about 145 minutes, about 150 minutes, about 155 minutes, about 160 minutes, about 165 minutes, about 170 minutes, about 175 minutes, about 180 minutes, about 185 minutes, about 190 minutes, about 195 minutes, about 200 minutes, about 205 minutes, about 210 minutes, about 215 minutes, about 220 minutes, about 225 minutes, about 230 minutes, about 235 minutes, about 240 minutes, about 245 minutes, or about 250 minutes.

In some embodiments, spatially tagging the genomic DNA can be performed by insertion of the transposon sequence into the genomic DNA with adapters described herein. An amplification step can be performed with primers to the adapters (e.g., inserted adapters into the genomic DNA). The amplified products can contain accessible genomic DNA which can be spatially tagged by methods described herein.

In some embodiments, spatially tagging the genomic DNA can be performed by transposome complexes immobilized on the surface of the substrate. In some embodiments, spatially tagging the genomic DNA can be performed by transposome complexes immobilized on a feature (e.g., a bead). In some embodiments, the transposome complexes are assembled prior to adding the biological sample to the substrate or features. In some embodiments, the transposome complexes are assembled after adding the biological sample to the substrate or features on a substrate. For example, a spatially barcoded substrate (e.g., array) can include a plurality of capture probes that include a Mosaic End sequence (e.g., a transposase recognition sequence). The Mosaic End sequence can be at the 3' end of the capture probe (e.g., the capture probe is immobilized by its 5' end and the Mosaic End sequence is at the 3' most end of the capture probe). The Mosaic End sequence can be a Mosaic End sequence for any of the transposase enzymes described herein. The Mosaic End sequence (e.g., a transposase recognition sequence) can be hybridized to a reverse complement sequence (e.g., oligonucleotide). For example, the reverse complement sequence (e.g., reverse complement to the Mosaic End sequence) can hybridize to the Mosaic End sequence thereby generating a portion of a double stranded DNA on the capture probe. The reverse complement to the Mosaic End sequence (e.g., oligonucleotide) can be provided to the spatially barcoded array prior to the biological sample being provided to the substrate. In some embodiments, the reverse complement to the Mosaic End sequence can be provided after the biological sample has been provided to the substrate. Transposase enzymes can be provided to the substrate and assemble at the double stranded portion of the capture probe (e.g., reverse complement oligonucleotide and the Mosaic End sequence hybridized to each other) thereby generating a transposome complex. For example, a transposome homodimer can be formed at the double stranded portion of the capture probe. A biological sample can be provided to the substrate such that the position of the capture probe on the substrate can be correlated with a position (e.g., location) in the biological sample. The transposome complexes can fragment (e.g., tagment) and spatially tag the genomic DNA.

In some embodiments, spatially tagging genomic DNA can be performed by hybridizing a single stranded capture probe to the tagmented DNA. In some embodiments the single stranded capture probe can be a degenerate sequence. In some embodiments, the single stranded capture can be a random sequence. The single stranded capture probe can have a functional domain, a spatial barcode, a unique molecular identifier, a cleavage domain, or combinations thereof. The single stranded capture probe (e.g., random sequence, degenerate sequence) can non-specifically hybridize tagmented genomic DNA, thereby spatially capturing the tagmented DNA. Methods for extension reactions are known in the art and any suitable extension reaction method described herein can be performed.

### Splint Oligonucleotides

As used herein, the term "splint oligonucleotide" refers to an oligonucleotide that, when hybridized to other polynucleotides, acts as a "splint" to recruit and position polynucleotides next to one another so that they can be ligated together. In some embodiments, the splint oligonucleotide is DNA or RNA. The splint oligonucleotide can include a nucleotide sequence that is partially complementary to nucleotide sequences from two or more different oligonucleotides. In some embodiments, the splint oligonucleotide assists in ligating a "donor" oligonucleotide and an "acceptor" oligonucleotide. In some embodiments, an RNA ligase, a DNA ligase, or other ligase can be used to ligate two nucleotide sequences together.

In some embodiments, the splint oligonucleotide can be between about 10 and about 50 nucleotides in length, e.g., between about 10 and about 45, about 10 and about 40, about 10 and about 35, about 10 and about 30, about 10 and about 25, or about 10 and about 20 nucleotides in length. In some embodiments, the splint oligonucleotide can be between about 15 and about 50, about 15 and about 45, about 15 and about 40, about 15 and about 35, about 15 and about 30, about 15 and about 30, or about 15 and about 25 nucleotides in length. In some embodiments, the fragmented DNA can include a sequence that is added (e.g., ligated) during fragmentation of the DNA. For example, during a transposition event (e.g., a Tn5 transposition event) an additional sequence (e.g., transposon end sequences) can be attached (e.g., covalently attached, e.g., via a ligation event) to the fragmented DNA (e.g., fragmented genomic DNA, e.g., tagmented genomic DNA). In some embodiments, the splint oligonucleotide can have a sequence that is complementary (e.g., a capture domain) to the fragmented DNA (e.g., fragmented genomic DNA, e.g., fragmented genomic DNA that includes a sequence that is added during fragmentation of the DNA, e.g. a first adapter attached during fragmentation of the DNA, e.g., a transposon end sequence) and a sequence that is complementary to the capture domain of the capture probe. In some embodiments, the splint oligonucleotide can be viewed as part of the capture probe. For example, the capture probe can be partially double stranded where a portion of the capture probe can function as a splint oligonucleotide that hybridizes to a portion of the capture probe (e.g., dsDNA portion) and can have a single strand portion that can hybridize to (e.g., capture domain) the fragmented DNA (e.g., fragmented genomic DNA e.g., tagmented, e.g., an adapter attached during fragmentation of the DNA, e.g., a Nextera adapter). The first adapter sequence (e.g., the sequence attached to the fragmented DNA complementary to the capture domain, e.g., Nextera adapter) can be any suitable sequence. In some embodiments, the adapter sequence can be between about 15 and 25 nucleotides long. In some embodiments, the adapter sequence can be about 16, about 17, about 18, about 19, about 20, about 21, about 22, about 23, or about 24 nucleotides long.

In some embodiments, a splint oligonucleotide can include a sequence that is complementary (e.g., capture domain) to the first adapter attached to the fragmented DNA (e.g., tagmented DNA). In some embodiments, the splint oligonucleotide includes a sequence that is not perfectly complementary to the first adapter (e.g., Nextera adapter) attached to the fragmented DNA (e.g., tagmented DNA), but is still capable of hybridizing the first adapter sequence (e.g., sequence complementary to the capture domain) ligated on to the fragmented DNA (e.g., Nextera adapter).

Any of a variety of capture probes having capture domains that hybridize to a splint oligonucleotide can be used in accordance with materials and methods described herein. As described herein, a capture domain is a domain on a capture probe capable of hybridizing the splint oligonucleotide to form a partially double stranded capture probe. For example, a single stranded capture probe can have a sequence complementary (e.g., capture domain) to a portion of the splint oligonucleotide, such that a partially double stranded capture probe is formed with a single stranded portion capable of hybridizing to the inserted transposon end sequences. In some embodiments, a splint oligonucleotide includes a sequence that is complementary (e.g., at least partially complementary) to the capture domain of the capture probe.

In some embodiments, the splint oligonucleotide includes a sequence that is not perfectly complementary to the capture domain of the capture probe, but is still capable of hybridizing the capture domain of the capture probe. In some embodiments, the splint oligonucleotide can hybridize to both the transposon end sequence, e.g., additional sequence attached to the tagmented DNA via and the capture domain of the capture probe via its sequence complementary to the capture domain. In such embodiments, where the splint oligonucleotide can hybridize to both the transposon end sequence (e.g., Nextera adapter, additional sequence attached to the fragmented DNA e.g., tagmented DNA), and the capture domain of the capture probe, the splint oligonucleotide can be viewed as part of the capture probe.

In some embodiments, the splint oligonucleotide can have a capture domain that is homopolymeric. For example, the capture domain can be a poly(T) capture domain.

In some embodiments, a splint oligonucleotide can facilitate ligation of the tagmented DNA and the capture probe. Any variety of suitable ligases known in the art or described herein can be used. In some embodiments, the ligase is T4 DNA ligase. In some embodiments, the ligation reaction can last for about 1 to about 5 hours. In some embodiments, the ligation reaction can last for about 2, about 3, or about 4 hours. In some embodiments, after ligation, strand displacement polymerization can be performed. In some embodiments, a DNA polymerase can be used to perform the strand displacement polymerization. In some embodiments, the DNA polymerase is DNA polymerase I.

### Multiplex Analysis

The present disclosure describes methods for permeabilizing biological samples under conditions sufficient to allow tagmentation of genomic DNA. The tagmented DNA can be captured via a capture probe (e.g., a capture probe and a splint oligonucleotide), however, at times it can be useful to simultaneously capture tagmented DNA and other nuclei acids (e.g., mRNA). For example, expression profiles of transcripts can be correlated (or not) with open chromatin. Put another way, the presence of transcripts can correlate with open chromatin (e.g., accessible chromatin) corresponding to the genes (e.g., genomic DNA) from which the transcripts were transcribed.

The present disclosure describes methods regarding the simultaneous capture of tagmented DNA and mRNA on spatially barcoded arrays. For example, a spatially barcoded array can have a plurality of capture probes immobilized on a substrate surface. Alternatively, a spatially barcoded array can have a plurality of capture probes immobilized on a feature. In some embodiments, the feature with a plurality of capture probes can be on a substrate. The capture probes can have spatial barcodes corresponding to a position (e.g., location) on the substrate. In some embodiments, the capture probes can further have a unique molecular identifier, one or more functional domains, and a cleavage domain, or combinations thereof. In some embodiments, the capture probe includes a capture domain. In some embodiments, the capture probe can be a homopolymeric sequence. For example, in a non-limiting way, the homopolymeric sequence can be a poly(T) sequence. In some embodiments, nucleic acid (e.g., mRNA) can be captured by the capture domain by binding (e.g., hybridizing) of poly(A) tails of mRNA transcripts. In some embodiments, tagmented DNA) can be captured by the capture domain of the capture probe by binding (e.g., hybridizing) a poly(A) tailed tagmented DNA. For example, after fragmenting the genomic DNA, gap filing (e.g., no strand displacement) polymerases and ligases can repair gaps and ligate breaks in the tagmented DNA. In some embodiments, a sequence complementary to the capture domain can be introduced to the fragmented DNA. For example, a poly(A) tail can be added to the tagmented DNA, such that the capture domain (e.g., poly(T) sequence) of the capture probe can bind (e.g., hybridize) to the poly(A) tailed tagmented DNA (See, e.g., WO 2012/140224, which is incorporated herein by reference). In some embodiments, a poly(A) tail is added to the tagmented DNA by a terminal transferase enzyme. In some embodiments, the terminal transferase enzyme is a terminal deoxynucleotidyl transferase (TdT), or a mutant variant thereof. TdT is an independent polymerase (e.g., it does not require a template molecule) that can catalyze the addition of deoxynucleotides to the 3' hydroxyl terminus of DNA molecules. Other template independent polymerases are known in the art. For example, Polymerase θ, or a mutant variant thereof, can be used as a terminal transferase enzyme (See, e.g., Kent, T., Polymerase θ is a robust terminal transferase that oscillates between three different mechanisms during end-joining, *eLIFE*, 5: e13740 doi: 10.7554/eLife. 13740, (2016)). Other methods of introducing a poly(A) tail are known in the art. In some embodiments, a poly(A) tail can be introduced to the tagmented DNA by a non-proofreading polymerase. In some embodiments, a poly(A) tail can be introduced to the fragmented DNA by a polynucleotide kinase.

In some embodiments, the TDT enzyme will generate tagments with a 3' poly(A) tail, thereby mimicking the poly(A) tail of an mRNA. In some embodiments, the capture domain (e.g., poly(T) sequence) of the capture probe would interact with the poly(A) tail of the mRNA and the generated (e.g., synthesized) poly(A) tail added to the fragmented (e.g., tagmented) DNA, thereby simultaneously capturing the fragmented DNA (e.g., tagmented DNA) and the mRNA transcript. The generated (e.g., synthesized) poly(A) tail on the fragmented DNA (e.g., tagmented DNA) could be between about 10 nucleotides to about 30 nucleotides long. The generated (e.g., synthesized) poly(A) tail on the fragmented DNA (e.g., tagmented DNA) could be about 11, about 12, about 13, about 14, about 15, about 16, about 17, about 18, about 19, about 20, about 21, about 22, about 23, about 24, about 25, about 26, about 27, about 28, or about 29 nucleotides long.

Additionally or alternatively, instead of a sequential (e.g. two-step) reaction (e.g., gap filling and ligating, followed by a terminal transferase) the fragmented (e.g., tagmented) DNA can be contacted with a polymerase. For example, the polymerase may be a DNA polymerase that may perform an extension reaction on the fragmented (e.g., tagmented DNA). Any variety of DNA polymerases known in the art or described herein can be used. The extended products can be captured and processed (e.g., amplified and sequenced) by any method described herein.

Post-hybridization steps are identical as described in Ståhl P.L., et al., Visualization and analysis of gene expression in tissue sections by spatial transcriptomics *Science*, vol. 353, 6294, pp. 78-82 (2016), which in incorporated herein by reference).

### Compositions

Also provided herein are compositions including capture probes, tagmented DNA, splint oligonucleotides, and one or more polymerases. In some embodiments, a splint oligonucleotide is hybridized to the capture domain of a capture probe. In some embodiments, a splint oligonucleotide is hybridized to a transposon end sequence of fragmented (e.g., tagmented) genomic DNA. In some embodiments, a splint oligonucleotide is hybridized to the captured domain of a capture probe and a transposon end sequence of fragmented genomic DNA. In some embodiments, the composition comprises one or more transposon end sequences. In some embodiments, one or more transposon end sequences are ligated to the capture probe. In some embodiments, one or more transposon sequences are released from the fragmented DNA either before or after ligation of the fragmented genomic DNA to the capture probe. In some embodiments, the composition includes a ligase (e.g., T4 DNA ligase). In some embodiments, the composition includes a gap filling polymerase. In some embodiments, the composition includes a DNA polymerase. In some embodiments, the composition includes one or more transposases. In some embodiments, the composition comprises transposome complexes.

In some embodiments, the capture domain of the capture probe binds the transposon end (e.g., without the facilitation by a splint oligonucleotide). In some embodiments, the composition includes a strand-displacing polymerase. In some embodiments, the composition includes a gap filling polymerase.

### qPCR and Analysis

Also provided herein are methods and materials for quantifying capture efficiency. In some embodiments, quantification of capture efficiency includes quantification of captured fragments (e.g., genomic DNA fragments, e.g., tagmented DNA fragments) from any of the spatial analysis methods described herein. In some embodiments, quantification includes PCR, qPCR, electrophoresis, capillary electrophoresis, fluorescence spectroscopy and/or UV spectrophotometry. In some embodiments, qPCR includes intercalating fluorescent dyes (e.g., SYBR green) and/or fluorescent labeled-probes (e.g., without limitation, Taqman probes or PrimeTime probes). In some embodiments, a NGS library quantification kit is used for quantification. For example, quantification can be performed using a KAPA library quantification kit (KAPA Biosystems), qPCR NGS Library Quantification Kit (Agilent), GeneRead Library Quant System (Qiagen), and/or PerfeCTa NGS Quantification Kit (Quantabio). In some embodiments that use qPCR for quantification, qPCR can include, without limitation, digital PCR, droplet digital (ddPCR), and ddPCR-Tail. In some embodiments that use electrophoresis for quantification, electrophoresis can include, without limitation, automated electrophoresis (e.g., TapeStation System, Agilent, and/or Bioanalzyer, Agilent) and capillary electrophoresis (e.g., Fragment Analyzer, Applied Biosystems). In some embodiments that use spectroscopy for quantification, the spectroscopy can include, without limitation, fluorescence spectroscopy (e.g., Qubit, Thermo Fisher). In some embodiments, NGS can be used to quantify capture efficiency.

In some embodiments, quantitative PCR (qPCR) is performed on the captured tagments. In some embodiments, the fragmented (e.g., tagmented) DNA is amplified, by any method described herein, before capture. For example, after capture of the fragmented DNA (e.g., tagmented DNA), ligation and strand displacement hybridization qPCR can be performed. In some embodiments, a DNA polymerase can be used to perform the strand displacement polymerization. Any suitable strand displacement polymerase known in the art can be used. In some embodiments, the DNA polymerase is DNA polymerase I. As exemplified in the Examples, DNA polymerase I can be incubated for strand displacement of the fragmented DNA (e.g., tagmented DNA) with reagents (e.g., BSA, dNTPs, buffer). In some embodiments, DNA polymerase I can be incubated with reagents on the substrate (e.g., on a feature e.g., a well) for about 30 minutes to about 2 hours. In some embodiments, DNA polymerase I can be incubated with reagents on the substrate for about 40 minutes, about 50 minutes, about 60 minutes, about 70 minutes, about 80 minutes, about 90 minutes, about 100 minutes, or about 110 minutes. In some embodiments, DNA polymerase I can be incubated with reagents on the substrate (e.g., on a feature e.g., a well) at about 35°C to about 40°C. In some embodiments, DNA polymerase I can be incubated with reagents on the substrate at about 36°C, about 37°C, about 38°C, or about °C, or about 39°C. In some embodiments, DNA polymerase I can be incubated with reagents on the substrate for about 1 hour at about 37°C.

After strand displacement hybridization is complete a qPCR reaction can be performed. In some embodiments, the capture probes ligated to the fragmented DNA (e.g., tagmented DNA), can be released from the surface of the substrate (e.g., feature). In some embodiments, a solution (e.g., release mix) can be incubated with the substrate to release the capture probes from the surface of the substrate. The release mix can contain reagents (e.g., BSA, enzymes, buffer, etc.). Methods of releasing capture probes from the substrate (e.g., a feature) are described herein. In some embodiments, an enzyme can cleave the capture probe. In some embodiments, the enzyme can be USER (uracil-specific excision reagent) enzyme. In some embodiments, the USER enzyme can be incubated with reagents on the substrate (e.g., a feature e.g., a well) for about 30 minutes to about 2 hours. In some embodiments, the USER enzyme can be incubated with reagents on the substrate for about 40 minutes, about 50 minutes, about 60 minutes, about 70 minutes, about 80 minutes, about 90 minutes, about 100 minutes, or about 110 minutes. In some embodiments, the USER enzyme with reagents on the substrate (e.g., a feature e.g., a well) at about 35°C to about 40°C. In some embodiments, the USER enzyme can be incubated with reagents on the substrate at about 36°C, about 37°C, about 38°C, or about 39°C. In some embodiments, the USER enzyme can be incubated with reagents on the substrate for about 1 hour at about 37°C.

After incubation with the USER enzyme, the samples (e.g., released capture probes ligated to fragmented DNA (e.g., tagmented DNA) in release mix, or a portion thereof) can be collected. In some embodiments, the sample volume can be reduced. Methods of reducing sample volume are known in the art and any suitable method can be used. In some embodiments, sample volume reduction can be performed with a Speed Vacuum (e.g., a SpeedVac). In some embodiments, the sample volume reduction can be about 50%, about 55%, about 60%, about 65%, about 70%, about 75%, about 80%, about 85%, or about 90% sample volume reduction. In some embodiments, the sample volume reduction can be about between 80% and 90% sample volume reduction. In some embodiments, the sample volume reduction can be about 81%, about 82%, about 83%, about 84%, about 85%, about 86%, about 87%, about 88, or about 89% sample volume reduction. In some embodiments, the sample volume reduction can be about 85% (e.g., about 10 µL after sample volume reduction).

In some embodiments, a qPCR reaction can be performed with the reduced sample volume. As described herein, any suitable method of qPCR can be performed. In some embodiments, a 1xKAPA HiFi HotStart Ready, 1x EVA green, and primers can be used. Amplification can be performed according to known methods in the art. For example, amplification can be performed accordingly: 72°C for 10 minutes, 98°C for 3 minutes, followed by cycling at 98°C for 20 seconds, 60°C for 30 seconds and 72°C for 30 seconds.

In some embodiments, one or more primer pairs can be used during the qPCR reaction. In some examples, a primer pair can cover the ligated portion (e.g., ligation site where the capture probe and adapter sequence (e.g., attached sequence to the fragmented DNA e.g., tagmented DNA)). For example, a primer pair covers the ligated portion and the capture probe. An amplification product will only be detected if ligation, and not just hybridization has occurred. In some embodiments, a different primer can cover the fragmented DNA (e.g. tagmented DNA) only. In some embodiments, the primer pair that covers the fragmented DNA (e.g., tagmented DNA) only can be a control for ligation. In some embodiments, qPCR can be performed with any of labeled nucleotides described herein.

In some embodiments, the samples can be purified. In some embodiments, the samples can be purified according to Lundin et al., Increased Throughput by Parallelization of Library Preparation for Massive Sequencing, PLOS ONE, 5(4), doi.org/10.1371/journal.pone.0010029 (2010), which is herein incorporated by reference.

In some embodiments, the average length of the captured fragmented DNA (e.g., tagmented DNA) can be determined. In some embodiments, a bioanalyzer (e.g., a 2100 Bioanalyzer (Agilent)) can be used. Any suitable bioanalyzer known in the art can be used. In some embodiments, qPCR and bioanalyzer analysis can be done on whole genomes (e.g., purified fragmented DNA e.g., tagmented DNA). In some embodiments, the qPCR and bioanalyzer analysis can be done on an immobilized biological sample (e.g., a fixed biological sample). For example, the methods described herein (e.g., pre-permeabilization, permeabilization) can be performed to capture fragmented DNA (e.g., tagmented DNA) and to optimize qPCR and bioanalyzer analysis for different biological samples.

In some embodiments, after ligation, a surface based denaturation step can be performed. Put another way, after ligation of the fragmented DNA (e.g., tagmented DNA) to the capture probe, followed by strand displacement hybridization described herein (e.g., DNA Polymerase I), a surface based denaturation step can be performed in a parallel workstream. In some embodiments, a basic solution can perform the surface based denaturation. For example, the basic solution can denature the captured double stranded fragmented DNA (e.g., tagmented DNA), thus generating captured single stranded capture probes ligated to fragmented DNA (e.g., tagmented DNA). In some embodiments, the basic solution is KOH. In some embodiments, the basic solution is NaOH. In some embodiments, the basic solution can be about 1M NaOH. Other basic solutions can be used in the methods described herein. In some embodiments, the basic solution can be applied for about 1 minute to about 1 hour. In some embodiments, the basic solution can be applied for about 10, about 20, about 30, about 40, or about 50 minutes. In some embodiments, the basic solution can be applied for about 1 to about 20 minutes. In some embodiments, the basic solution about be applied for about 2, about 3, about 4, about 5, about 6, about 7, about 8, about 9, about 10, about 11, about 12, about 13, about 14 about 15, about 16, about 17, about 18, or about 19 minutes. In some embodiments, the basic solution can be applied at a temperature of between about 30°C to about 40°C. In some embodiments, the basic solution can be applied at about 31°C, about 32°C, about 33°C, about 34°C, about 35°C, about 36°C, about 37°C, about 38°C, or about 39°C. In some embodiments, the basic solution can be applied for about 10 minutes at about 37°C.

In some embodiments, the denaturation step can expose the fragmented DNA (e.g., tagmented DNA) to hybridization by a probe. In some embodiments, the probe can be an oligonucleotide probe. In some embodiments, the oligonucleotide probe can have a detectable label (e.g., any of the variety of detectable labels described herein). In some embodiments, the detectable label can be Cy5. In some embodiments, the oligonucleotide probe can be Cy5 labeled. In some embodiments, the Cy5 labeled oligonucleotide probe can hybridize to a complementary sequence in the fragmented DNA (e.g., tagmented DNA). In some embodiments, the Cy5 labeled oligonucleotide can hybridize to the sequence attached (e.g., Nextera adapter, e.g., first adapter or second adapter) to the fragmented DNA (e.g., tagmented DNA). In some embodiments, the Cy5 label can be detected. For example, detecting the Cy5 label in the oligonucleotide probe can reveal the spatial location of the DNA tagments. In some embodiments, the biological sample can be stained (e.g., hematoxylin and eosin stain). Methods of staining a biological sample are known in the art and described herein. In some embodiments, the biological sample can be imaged.

### Whole Genome Analysis

Whole genome analysis (e.g., spatial genomics) can also be performed on biological samples. For example, the spatial ATAC methods described herein are designed to capture accessible (e.g., "open" or transcriptionally active) areas of a genome, however, spatially capturing the whole genome (e.g., DNA) is also possible. To enable capture of the whole genome, chromatin structure is disrupted by degradation of the histones

Thus provided herein are methods for determining the location of DNA in a biological sample, the method including: (a) a biological sample on an array including a plurality of capture probes, where a capture probe of the plurality of capture probes includes: (i) a spatial barcode and (ii) a capture domain; (b) contacting the biological sample with a protease, where the protease is capable of degrading one or more histone proteins, thereby releasing the DNA; (c) contacting a transposome to the biological sample to insert transposon end sequences into the released genomic DNA, thereby generated fragmented genomic DNA; (d) hybridizing a transposon end sequence of the fragmented DNA to the capture domain; (e) releasing transposon end sequences not bound to the capture domain; and (f) determining (i) a sequence of the spatial barcode or a complement thereof, and (ii) all or a portion of a sequence of the DNA, or a complement thereof, and using the determined sequences of (i) and (ii) to determine the location of DNA in the biological sample.

Thus, provided herein are methods that include treating a biological sample with a protease capable of degrading histone proteins resulting in the generation of fragmented genomic DNA. The fragmented genomic DNA can be captured by the capture domain of a capture probe, where, for example, the transposon end sequence inserted into the genomic DNA include a sequence complementary to a capture domain of a capture probe.. In some embodiments, the capture domain comprises a homopolymeric sequence. In some embodiments, the capture domain comprises a unique sequence.

In some embodiments, a biological sample is permeabilized by exposing the sample to a protease capable of degrading histone proteins. As used herein, the term "histone protein" typically refers to a linker histone protein (e.g., H1) and/or a core histone protein (e.g., H2A, H2B, H3, and H4). In some embodiments, a protease degrades linker histone proteins, core histone proteins, or linker histone proteins and core histone proteins. Any suitable protease capable of degrading histone proteins in a biological sample can be used. Non-limiting examples of proteases capable of degrading histone proteins include proteases inhibited by leupeptin and TLCK (Tosyl-L-lysyl-chloromethane hydrochloride), collagenase, a protease encoded by the EUO gene from *Chlamydia trachomatis serovar A*, granzyme A, a serine protease (e.g. trypsin or trypsin-like protease, neutral serine protease, elastase, cathepsin G), an aspartyl protease (e.g., cathepsin D), a peptidase family C1 enzyme (e.g. cathepsin L), a protease that is inhibited by the diazomethane inhibitor Z-Phe-Phe-CHN(2) or the epoxide inhibitor E-64, a lysosomal protease, or an azurophilic enzyme (e.g., cathepsin G, elastase, proteinase 3, neutral serine protease). In some embodiments, a serine protease is a trypsin enzyme, trypsin-like enzyme or a functional variant or derivative thereof (e.g., P00761; C0HK48; Q8IYP2; Q8BW11; Q6IE06; P35035; P00760; P06871; Q90627; P16049; P07477; P00762; P35031; P19799; P35036; Q29463; P06872; Q90628; P07478; P07146; P00763; P35032; P70059; P29786; P35037; Q90629; P35030; P08426; P35033; P35038; P12788; P29787; P35039; P35040; Q8NHM4; P35041; P35043; P35044; P54624; P04814; P35045; P32821; P54625; P35004; P35046; P32822; P35047; C0HKA5; C0HKA2; P54627; P35005; C0HKA6; C0HKA3; P52905; P83348; P00765; P35042; P81071; P35049; P51588; P35050; P35034; P35051; P24664; P35048; P00764; P00775; P54628; P42278; P54629; P42279; Q91041; P54630; P42280; C0HKA4) or a combination thereof. In some embodiments, a trypsin enzyme is P00761, P00760, Q29463, or a combination thereof. In some embodiments, a protease capable of degrading one or more histone proteins comprises an amino acid sequence with at least 80% sequence identity to P00761, P00760, or Q29463. In some embodiments, a protease capable of degrading one or more histone proteins comprises an amino acid sequence with at least 85%, 90%, 91%, 92%, 93%, 94%, 95%, 96%, 97%, 98%, or 99% identity to P00761, P00760, or Q29463. A protease may be considered a functional variant if it has at least 50% e.g. at least 60%, 70%, 80%, 90%, 95%, 96%, 97%, 98%, 99%, or 100% of the activity relative to the activity of the protease in condition optimum for the enzyme.

Additionally, the protease can be contained in a reaction mixture (solution), which also includes other components (e.g., buffer (e.g., Tris-HCl, salt, chelator (e.g., EDTA), detergent (e.g., SDS)). The reaction mixture may be buffered, having a pH of about 6.5-8.5, e.g. about 7.0-8.0. Additionally, the reaction mixture may be used at any suitable temperature, such as about 10-45°C, e.g. about 10-44°C, 11-43°C, 12-42°C, 13-41°C, 14-40°C, 15-39°C, 16-38, °C 17-37°C, e.g. about 10°C, 12°C, 15°C, 18°C, 20°C, 22°C, 25°C, 28°C, 30°C, 33°C, 35°C or 37 °C, preferably about 30-40°C, e.g. about 37°C.

In some embodiments, the reaction mixture can be incubated with the biological sample from about 1 minute to about 30 minutes, from about 5 minutes to about 25 minutes, from about 10 minutes to about 20 minutes, or about 15 minutes. In some embodiments, the reaction mixture is incubated with the biological sample for 37°C for about 10 minutes.

In some embodiments, the method includes imaging and/or staining the biological sample, such as for example, haematoxylin and eosin staining.

In some embodiments, the capture probe includes a cleavage domain, one or more functional domain, a unique molecular identifier, or combinations thereof.

In some embodiments, the method includes an active migration step wherein the fragmented genomic DNA is migrated to the array by applying an electric field.

In some embodiments, the hybridizing in step (d) comprises hybridizing the transposon end sequence, or a portion thereof, to the capture domain, or a portion thereof, of the capture probe. In some embodiments, the method extending a 3' end of the capture probe using the fragmented genomic DNA as a template. In some embodiments, the extending step is performed using a DNA polymerase having strand displacement activity. In some embodiments, the method includes gap filling (e.g., as described herein) between the transposon end sequence and the fragmented genomic DNA. In some embodiments, the transposome includes a transposase enzyme (e.g., any of the transposase enzymes described herein), such as a Tn5 transposase enzyme, a Mu transposase enzyme, a Tn7 transposase enzyme, a Vibrio species transposase, or functional derivative or variants thereof.

In some embodiments, the releasing in step (d) comprises heating the biological sample. In some embodiments, the heating includes heating to a temperature of about 65°C to 85°C.

In some embodiments, determining the location of DNA in a biological sample further includes spatially analyzing the whole genome of the biological sample.

### EXAMPLES

### Example 1. ATAC workflow for spatial arrays

### Tissue Preparation and spatial ATAC Workflow

Tissue samples are snap frozen in optimal cutting temperature (OCT) blocks, cryosectioned into 10 µm sections, placed on a Visium spatial array slide (10X Genomics, Inc.) and heated at 37°C for 1 minute. The tissues are formalin fixed (1% formaldehyde in PBS) at room temperature for 10 min and rinsed several times in PBS. Following fixation, isopropanol (80%) is flooded onto the slide, removed and the slide with the tissue is air-dried. The tissue is pre-permeabilized with collagenase in HBSS buffer (0.2 U collagenase, 0.2 mg BSA) at 37°C for 20 min and washed with a 0.1X SSC wash buffer. Following the wash, the tissue is permeabilized at RT for 10 min with a buffered solution containing 0.1% NP40, 0.1% Tween-20, and 0.01% digitonin.

Tagmentation reagents (Illumina, Inc.) are deposited on the permeabilized tissue for 1 hour at 37°C. The reagents include 2.5 µl Tn5 in tagmentation mix (1x TD buffer, 31% PBS, 0.01% digitonin, 0.1% Tween-20, H₂O). The slide is washed (Wash buffer 3) and tagmentation is stopped with Tagmentation Stop-buffer (0.01% SDS, 50 mM EDTA, 10 mM Tris-HCl, and H₂O) at 37°C for 10 minutes.

A 2µM splint oligonucleotide mixture (1x NEB 2.1, H₂O) and associated reagents are added to the tissue and incubated at 75°C for 15 minutes. The temperature is slowly decreased to 20°C overnight, with a ramp speed cooling of 0.1°C/min to enable tagment diffusion, hybridization of splint oligonucleotides to the tagments, and subsequent capture of the splint oligonucleotide-tagment complexes.

Polymerization and ligation are performed using a T4 DNA mix (5U T4 DNA polymerase, 0.4U T4 DNA ligase, 1 µM dNTPs, 3 mM ATP, H₂O, 1x NEB2.1), and incubated at 20°C for 3 hours. Tissue is removed using 2 mg/ml Proteinase K in PKD-buffer, and incubation is carried out at 56°C for 1 hour. The slides are washed for 10 minutes at 50°C in Wash buffer 1 (0.3x SSC, SDS) followed by washing with Wash buffer 2 (0.2x SSC) at room temperature for 1 minute, ending in Wash buffer 3 (0.1x SSC) and spin-drying.

### Library Preparation and Sequencing

To enable bulk library preparation of the spatially barcoded surface bound tagments, denaturation of the capture probes on the slides is performed with 0.08N KOH at room temperature for 10 minutes. The mixture is transferred from the slide to LoBind tubes containing 1M Tris at pH 7.0. The denaturation step is repeated and cleanup performed (MinElute Reaction Cleanup Kit, Qiagen) followed by elution in 20 µl of elution buffer. The samples are processed into sequencing libraries and sequenced on an Illumina Nextseq 500 instrument using paired end reads following manufacturer protocols.

### Staining Procedures

After tagmentation, a layer of 80% isopropanol is added to the tissues on the slides, removed, and air-dried as described above. The tissue sections are stained with hematoxylin at room temperature for 7 minutes, rinsed in water, incubated in bluing buffer at room temperature for 2 minutes, rinsed in water, and stained with eosin at room temperature for 10 or 20 seconds depending on the tissue sample (e.g., mouse brain and prostate cancer tissue, respectively). The slides are rinsed in water, spin-dried, covered with glycerol and a coverslip, and imaged.

### Immunohistochemistry

The tissue sections on the slides are blocked by incubation with staining buffer for 5 minutes. The staining buffer is removed and the primary antibody dilution added and incubated at room temperature for 30 minutes. Two washes are performed with staining buffer for 3 minutes each, followed by addition of the secondary antibody dilution, and incubation at room temperature for 15 minutes. Three washes are performed with staining buffer for 3 minutes each, and finally pipette washed with PBS one time. The slides are spin-dried, covered with glycerol and a coverslip, and imaged.

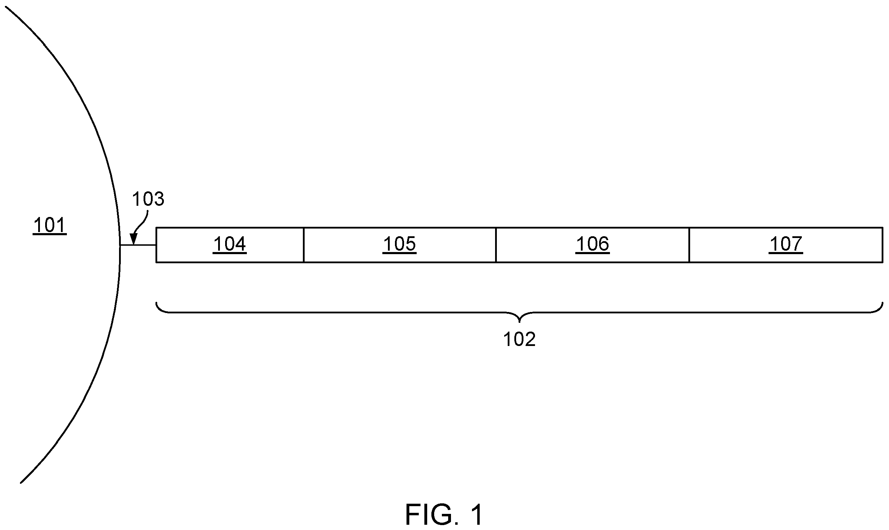

**FIG. 1** is a schematic diagram showing an example of a capture probe, as described herein. As shown, the capture probe **102** is optionally coupled to a feature **101** by a cleavable linker **103,** such as a photocleavable linker. The capture probe can include functional sequences that are useful for subsequent processing, such as functional sequence **104,** which can include a sequencer specific flow cell attachment sequence, e.g., a P5 or P7 sequence, as well as functional sequence **105,** which can include sequencing primer sequences, e.g., a R1 primer binding site, a R2 primer binding site. In some embodiments, sequence **104** is a P7 sequence and sequence **105** is a R2 primer binding site. A spatial barcode **106** can be included within the capture probe for use in barcoding the target analyte. The functional sequences can generally be selected for compatibility with any of a variety of different sequencing systems, e.g., Ion Torrent Proton or PGM, Illumina sequencing instruments, PacBio, Oxford Nanopore, etc., and the requirements thereof. In some embodiments, functional sequences can be selected for compatibility with non-commercialized sequencing systems. Examples of such sequencing systems and techniques, for which suitable functional sequences can be used, include (but are not limited to) Ion Torrent Proton or PGM sequencing, Illumina sequencing, PacBio SMRT sequencing, and Oxford Nanopore sequencing. Further, in some embodiments, functional sequences can be selected for compatibility with other sequencing systems, including non-commercialized sequencing systems.

In some embodiments, the spatial barcode **106,** functional sequences **104** (e.g., flow cell attachment sequence) and **105** (e.g., sequencing primer sequences) can be common to all of the probes attached to a given feature. The spatial barcode can also include a capture domain **107** to facilitate capture of a target analyte. In some embodiments, additional sequences can be included in the capture probe. For example, a unique molecular identifier can be included between the functional sequence **105** and the spatial barcode **106,** or a unique molecular identifier can be inserted between the spatial barcode **106** and the capture domain **107.** Additional functional sequences, for example primer sequencing (e.g., sequencing primer binding sequences, amplification primer binding sequences) can also be included in the capture probe, for example a second functional sequence that is different from the first functional sequence(s) can be found between the spatial barcode **106** and the capture domain **107.** In some embodiments, the capture probe can include one or both of a unique molecular identifier and a second functional sequence.

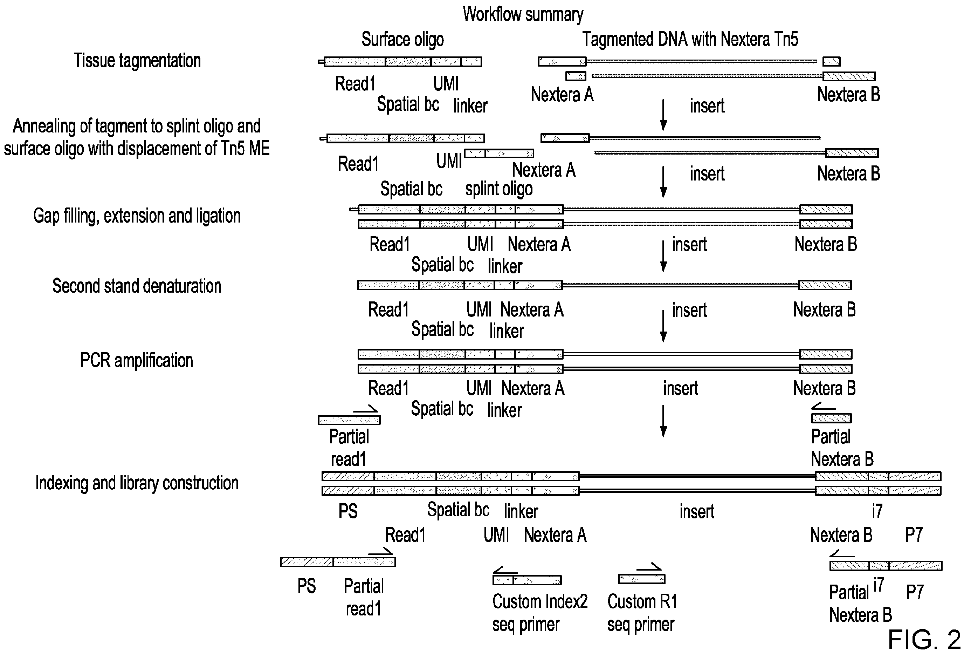

**FIG. 2** shows an exemplary spatial ATAC (spATAC) workflow. The workflow includes contacting a biological sample with an array comprising a plurality of capture probes and tagmenting accessible genomic DNA with a transposome resulting in fragmented DNA with transposon end sequences inserted (e.g., Nextera A, Nextera B). The resulting tagmented DNA results in a gap between transposon end sequences and the fragmented genomic DNA. In some examples, the transposon end sequences adjacent to the gap are released (e.g., removed) from the fragmented genomic DNA. In some examples, such transposon end sequences are released with a heat gradient. A splint oligonucleotide is hybridized to the capture domain of the capture probe, or a portion thereof, and the remaining transposon end sequence. Gap filling, DNA extension (e.g., extension with a DNA polymerase), and ligation of the transposon end sequence to the capture probe is performed. Gap filling polymerase fills the gap between the splint oligonucleotide and the fragmented genomic DNA (e.g., a portion of which included the released transposon end sequence) with the complementary nucleic acids of the gap needing to be filled. DNA extension results in generating a second strand with a sequence complementary to the spatial barcode and a double stranded DNA complex. In some examples, the generated second strand is released (e.g., denatured), collected, amplified, and processed for indexing and library construction. The resulting library is used to determine the location of the accessible chromatin in the biological sample.

### Example 2. Spatial ATAC Analysis

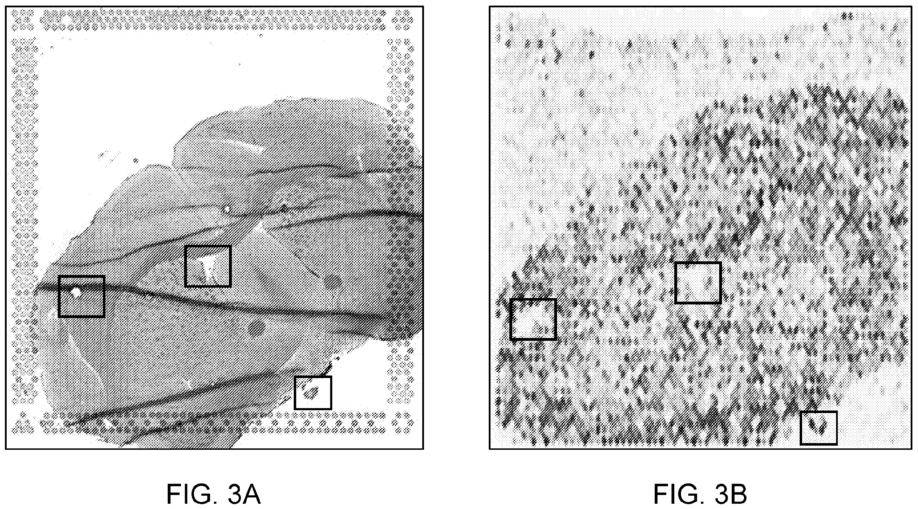

The following results were generated by the exemplary spATAC workflow described in Example 1. **FIG. 3A** shows a hematoxylin and eosin (H&E) stained mouse brain with a human glioma xenograft tissue section. **FIG. 3B** shows spatial clustering (e.g., spot positions) of the mouse brain tissue section and the data show a broad spatial agreement between captured genomic DNA and the morphological structure of the mouse brain tissue section. The boxes in both figures indicate areas that serve as internal quality control areas to demonstrate that spatial resolution of the spATAC methods was obtained (e.g., in two boxes there are no tissue and no clustering seen, in one box where there is a little piece of tissue there is clustering).

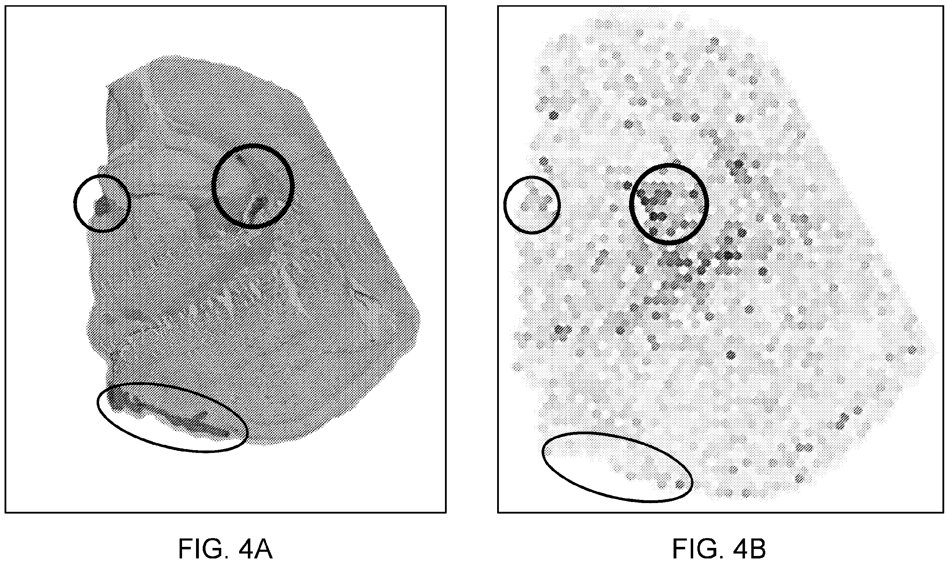

**FIGs. 4A-B** show representative pictures from an experiment with tissue where mouse expressed human cells are used to test spatial resolution of the workflow described in **FIG. 2****.** **FIG. 4A** shows an H&E stained mouse brain with human glioma xenograft tissue section. The darker portions circled on the image are areas of the tissue section with human cells. **FIG. 4B** shows identification of human DNA identified within the mouse tissue section indicated by circles. The data show that clustering reflects the morphology of the tissue section and is sensitive in detecting and distinguishing between human versus mouse DNA in the sample tissue section.

**Table 1. Reads per spot in natural logarithm scale**

| Index | Average | Std. |
|---|---|---|
| 1 | 14.197071531445 | 11.3092012410549 |
| 2 | 34.9050753875557 | 32.2738289613216 |
| 3 | 36.3514060287275 | 23.8985761010508 |
| 4 | 4.52903081732917 | 3.35271103443555 |
| 5 | 24.6123792800702 | 31.4306497970632 |
| 6 | 4.91824739513759 | 4.25066145637371 |

**Table 2. Genes per spot in natural logarithm scale**

| Index | Average | Std. |
|---|---|---|
| 1 | 12.8216514642343 | 9.68067308393996 |
| 2 | 22.8551709492461 | 14.954558667119 |
| 3 | 32.3910580619057 | 20.1521778991193 |
| 4 | 4.16033943724877 | 2.92892111421054 |
| 5 | 17.9218612818262 | 17.9319400455273 |
| 6 | 4.50601122094577 | 3.76867917965407 |

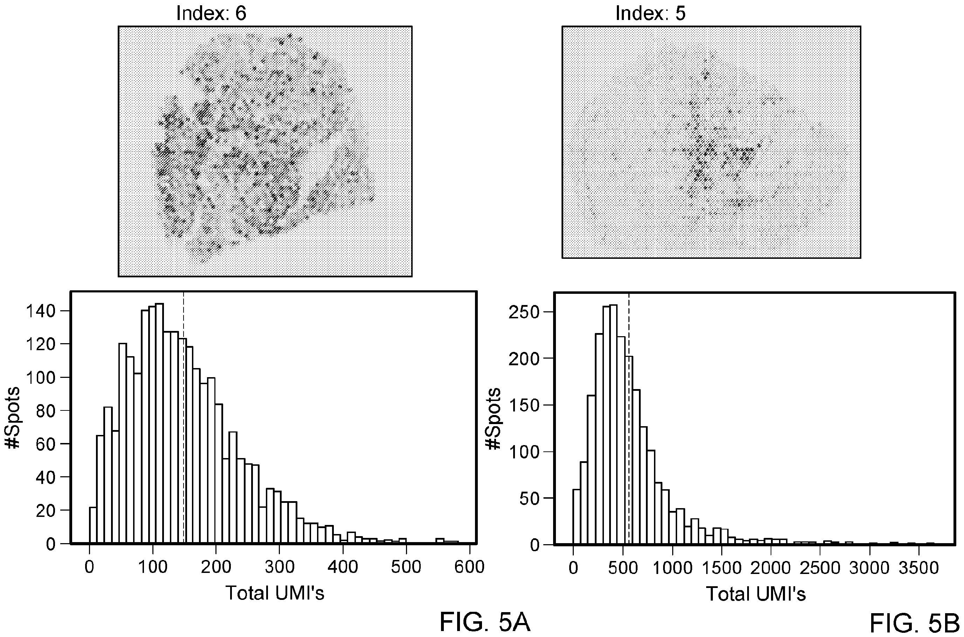

**FIGs. 5A-B** show replicate graphs of the number of spots by the total number of unique molecular identifiers (UMIs) identified in two mouse brain tissue sections (index 6 and index 5, respectively in Table 1 and Table 2; (index 1-4 data not shown)). The data shown were mapped to chromosomes rather than genes. The dashed line indicates the mean (**FIG. 5A-5B****,** bottom). Spot positions within the biological sample are shown for index 6 and 5 (**FIG. 5A-5B****,** top). Darker spots indicate more UMIs detected at that location.

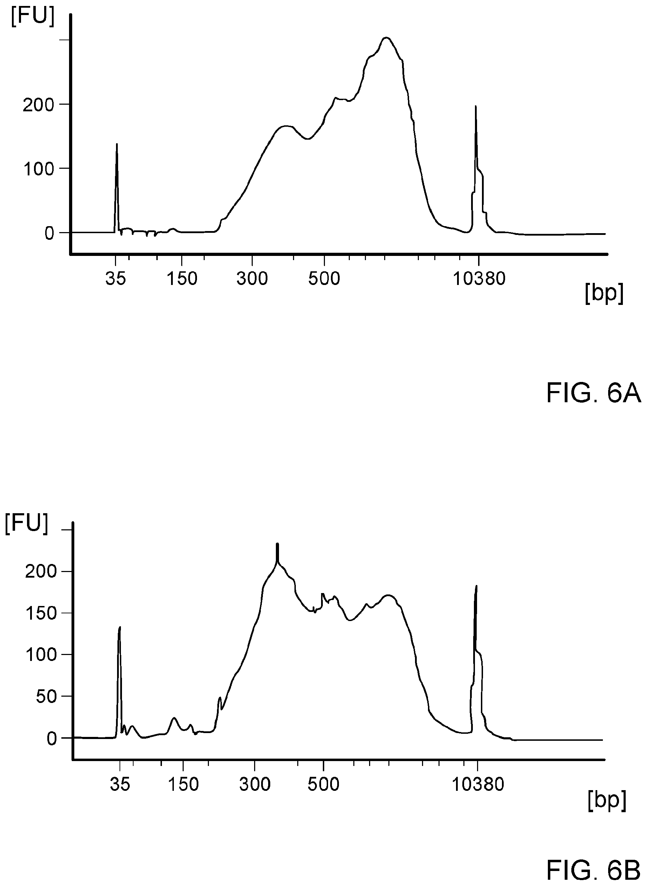

**FIGs. 6A-B** show graphs indicating recovery of nucleosome periodicity when the biological sample was not treated with Proteinase K after tagmentation and H&E staining was also performed after tagmentation. The data indicate that the chromatin structure stays intact without the Proteinase K treatment and staining post tagmentation.

Collectively, the data demonstrate that the exemplary spATAC workflow described in **FIG. 2** can be used to spatially assay for accessible genomic DNA from biological samples. Further, the method is also sensitive, since human and mouse DNA were separately identifiable by the workflow in mouse brain with a human glioma xenograft tissue section.

### Example 3. Whole genome analysis workflow

Whole genome analysis (e.g., spatial genomics) can also be performed on tissue samples. For example, the spatial ATAC methods described herein are designed to capture accessible (e.g., "open" or transcriptionally active) areas of a genome, however, spatially capturing the whole genome is also possible. To enable capture of the whole genome, chromatin structure is disrupted by degradation of the histones after the collagenase treatment as described in Example 1, followed by incubation with Proteinase K (2 mg/ml) in a Proteinase K-buffer (1% SDS, 50 mM EDTA, 10 mM Tris-HCl, H2O) at 37°C for 10 minutes. The resulting fragmented DNA can be captured on the spatial array followed by library preparation and sequence analysis as described herein.

### Example 4. Spatial ATAC Analysis

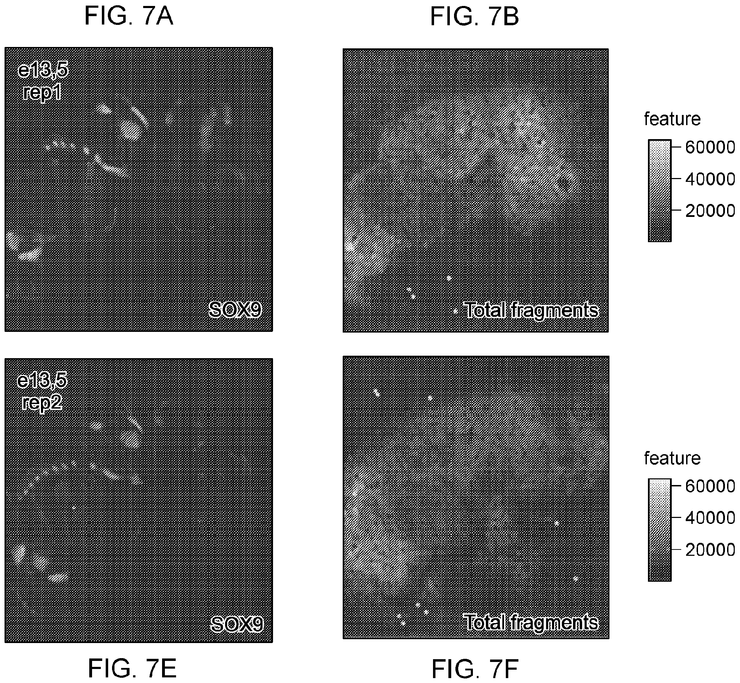

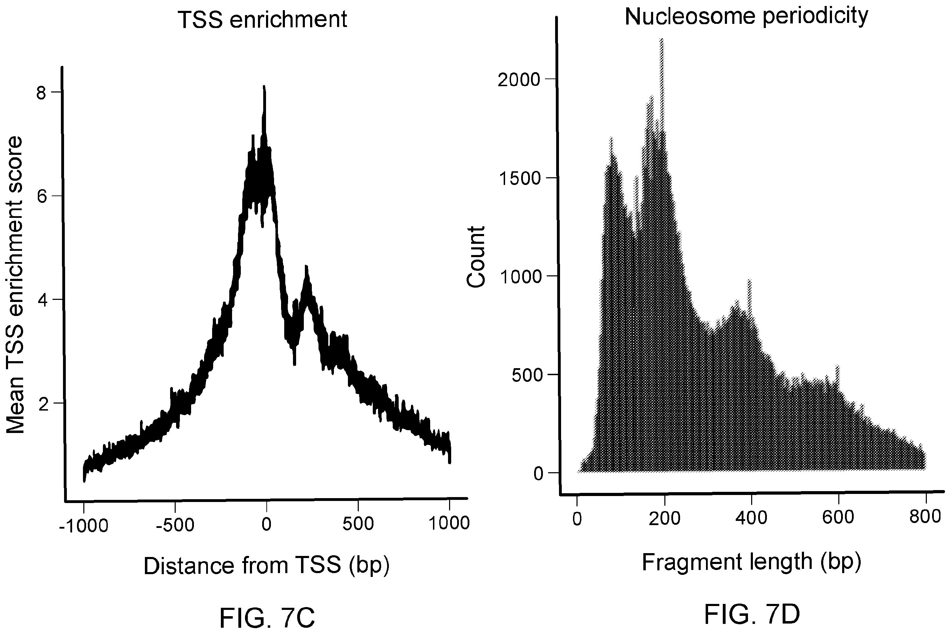

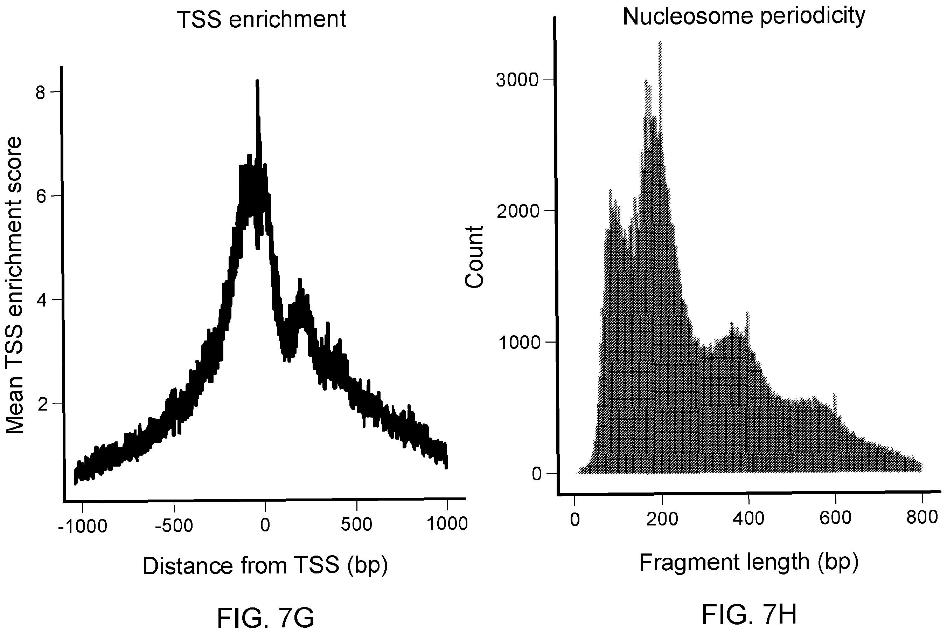

**FIGs. 7A-H** show replicate analysis performed on the two consecutive mouse embryo tissue sections immuno-stained with a SOX9 antibody (**FIGs. 7A** and **7E**) prior to the spatial ATAC-seq workflows described herein. **FIGs. 7B** and **7F** shows the total number of tagmented DNA fragments captured per spot which is indicative of the spatial resolution of fragmented genomic DNA capture. **FIGs. 7C** and **7G** are graphs showing the transcriptional start site (TSS) enrichment for the spots under the tissue sections for the consecutive sections and **FIGs. 7D** and **7H** are graphs showing the corresponding nucleosome periodicity reflected in the captured tagment size distribution reconstructed after sequencing.

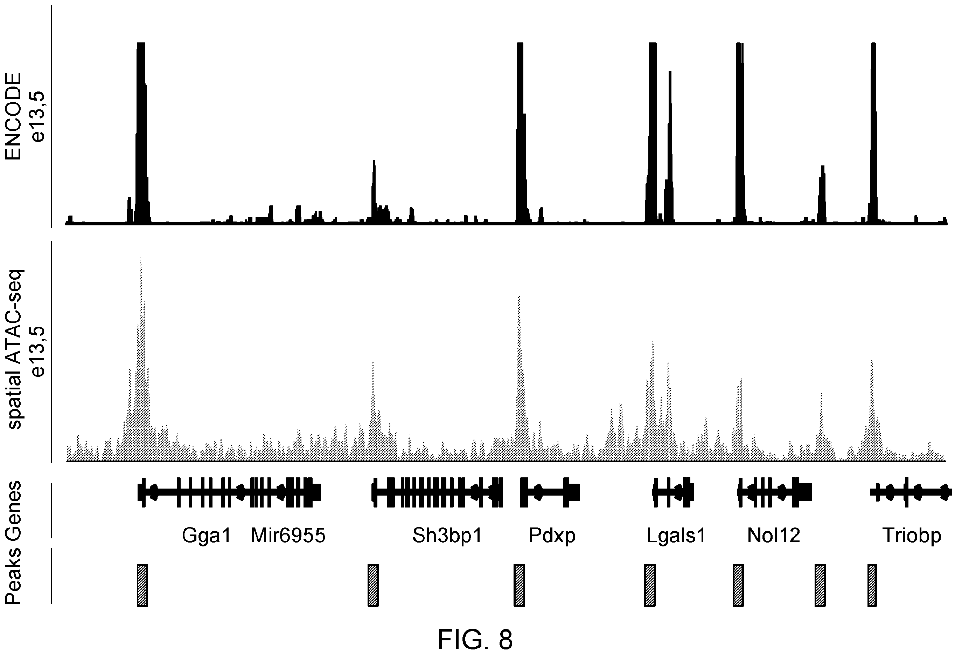

**FIG. 8** shows genome traces of ATAC-seq read densities for a reference mouse dataset (e13,5 ENCODE) and spatial ATAC-seq (e13,5) from the mouse embryos shown in **FIGs. 7A** and **7E****.** The spatial ATAC-seq signal enrichment and peak calling (bottom) show matching positions for fragment enrichment of the following genes: Gga1, Mir6955, Sh3bp1, Pdxp, Lgals1, Nol12, and Triobp, thus, demonstrating that the spatial ATAC workflows described herein can be used to detect accessible genomic DNA from biological samples (e.g., mouse embryo sections).

### Example 5. Spatial ATAC analysis

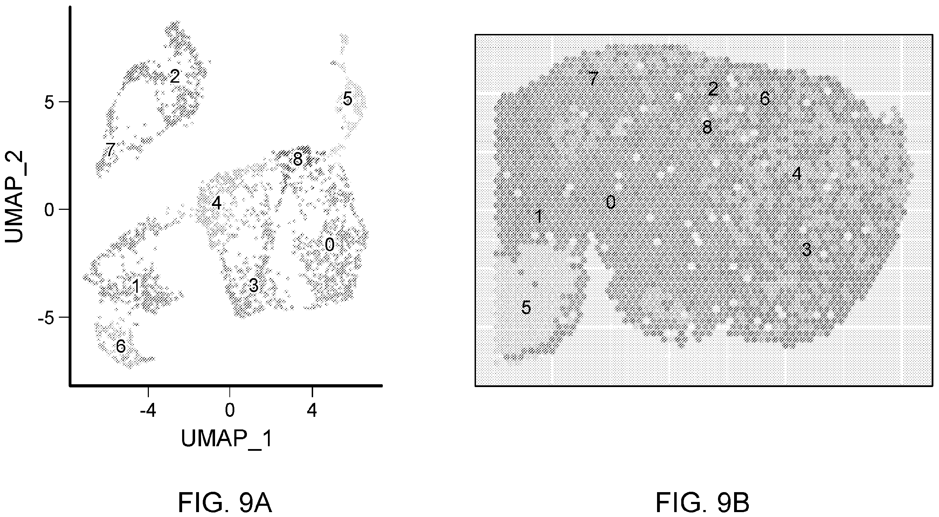

The data shown in **FIGs. 9A-12B** were generated according to the library preparation and sequencing step, staining procedures, and immunohistochemistry performed as described in Example 1. However, the tissue preparation and spatial ATAC workflow were prepared according to the protocol below.

### Tissue Preparation and spatial ATAC Workflow

Tissue samples are snap frozen in optimal cutting temperature (OCT) blocks, cryosectioned into 10 µm sections, placed on a Visium spatial array slide and heated at 37°C for 1 minute. The tissues are formalin fixed (1% formaldehyde in PBS) at room temperature for 10 min and rinsed several times in PBS. Following fixation, isopropanol (80%) is flooded onto the slide, removed and the slide with the tissue is air-dried. The tissue is pre-permeabilized with collagenase in HBSS buffer (0.2 U collagenase, 0.2 mg BSA) at 37°C for 20 min and washed with a 0.1X SSC wash buffer. Following the wash, the tissue is permeabilized at RT for 10 min with a buffered solution containing 0.1% NP40, 0.1% Tween-20, and 0.01% digitonin.

Tagmentation reagents (Illumina) are deposited on the permeabilized tissue for 1 hour at 37°C. The reagents included 2.5 µl Tn5 in tagmentation mix (1x TD buffer, 31% PBS, 0.01% digitonin, 0.1% Tween-20, H₂O). The slide is washed (Wash buffer 3) and tagmentation is stopped with Tagmentation Stop-buffer (0.01% SDS, 50 mM EDTA, 10 mM Tris-HCl, and H₂O) at 37°C for 10 minutes.

A 2µM splint oligonucleotide mixture in a salt solution (NEB 2.1 or SSC based) containing Proteinase K at 0.2 mg/µl and a dilution of Triton X-100 (0.2-0.01%) were added to the tissue section and incubated at 30°C for about 2-8 hours to enable tagment release via protease digestion, tagment diffusion, and hybridization of splint oligonucleotides to the tagments, and subsequent capture of the splint oligonucleotide-tagment complexes by the capture probes.

Polymerization and ligation are performed using a T4 DNA mix (5U T4 DNA polymerase, 0.4U T4 DNA ligase, 1 µM dNTPs, 3 mM ATP, H2O, 1x NEB2.1 supplemented with Triton X-100 (0.2-0.01%)), and incubated at 20°C for 3 hours. Tissue is removed using 2 mg/ml Proteinase K in PKD-buffer, and incubations carried out at 56°C for 1 hour. The slides are washed for 10 minutes at 50°C in Wash buffer 1 (0.3x SSC, SDS) followed by washing with Wash buffer 2 (0.2x SSC) at room temperature for 1 minute, ending in Wash buffer 3 (0.1x SSC) and spin-drying.

**FIG. 9A** is a uniform manifold approximation and projection (UMAP) of unbiased graph-based clustering and the cluster assignment of each spot in the tissue section is shown in **FIG. 9B. FIG. 9B** show a (UMAP) plots of gene accessibility across tissue spots in a mouse section processed according to spatial ATAC-seq. Collectively, the data show that spatial ATAC-seq captures meaningful biological variation in gene accessibility across tissue regions.

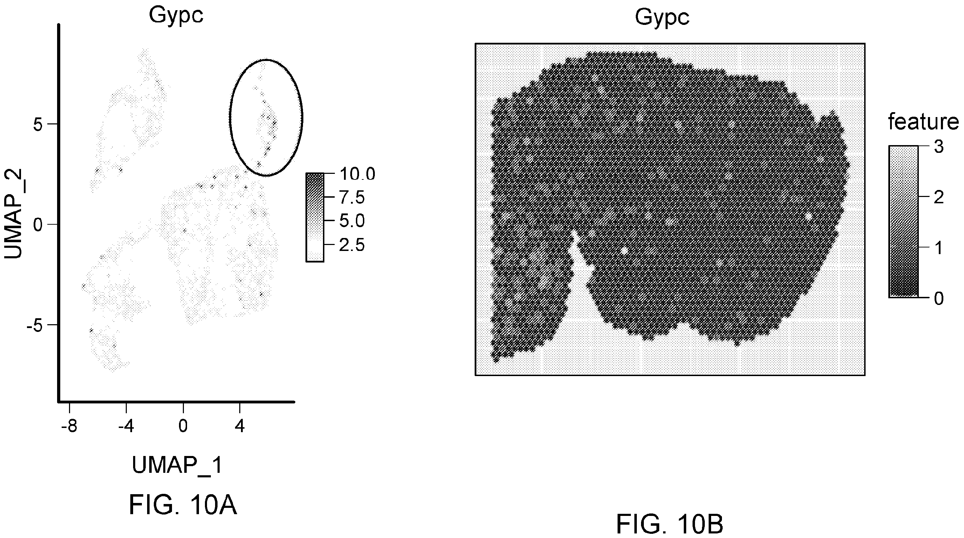

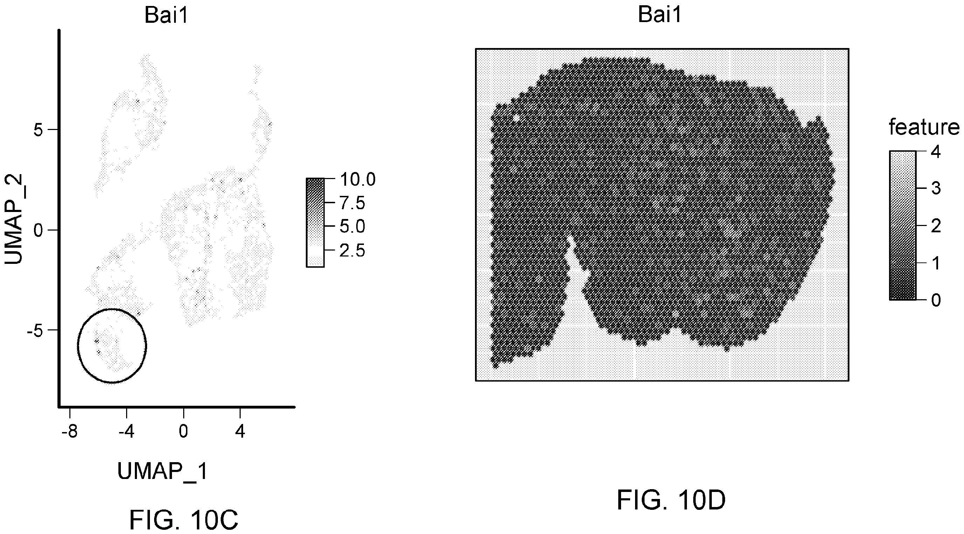

**FIGs. 10A-D** shows UMAP plots (**FIG. 10A** and **FIG. 10C**) colored by the relative accessibility of two gene regions, glycophorin C (Gypc) and adhesion G protein coupled receptor 1 (Bai1), found to be differentially accessible between regions of the tissue section which correspond to the relative accessibility shown in **FIG. 10B** and **FIG. 10D****.**

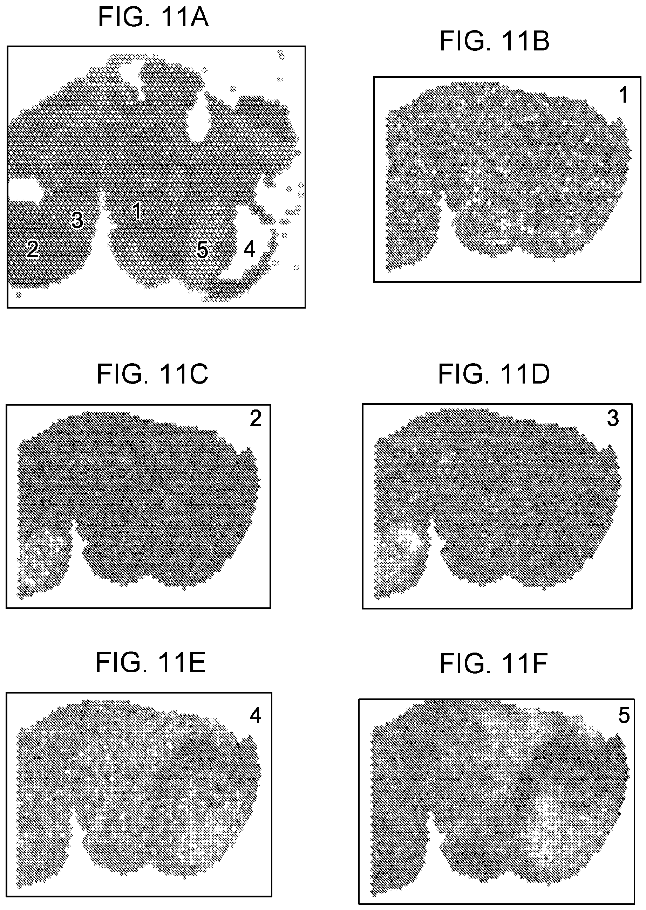

**FIG. 11A** shows clustering based on gene expression. Clusters, that is, the tissue regions with characteristic gene expression profiles are indicated by numbers. **FIGs. 11B-F** show the accessibility of some of the top marker genes (FIG. 11B 28 genes; FIG. 11C 61 genes; FIG. 11D 132 genes; FIG. 11E 180 genes; FIG. 11F 807 genes) for each cluster in an adjacent section according to spatial ATAC-seq, which manifests high concordance.

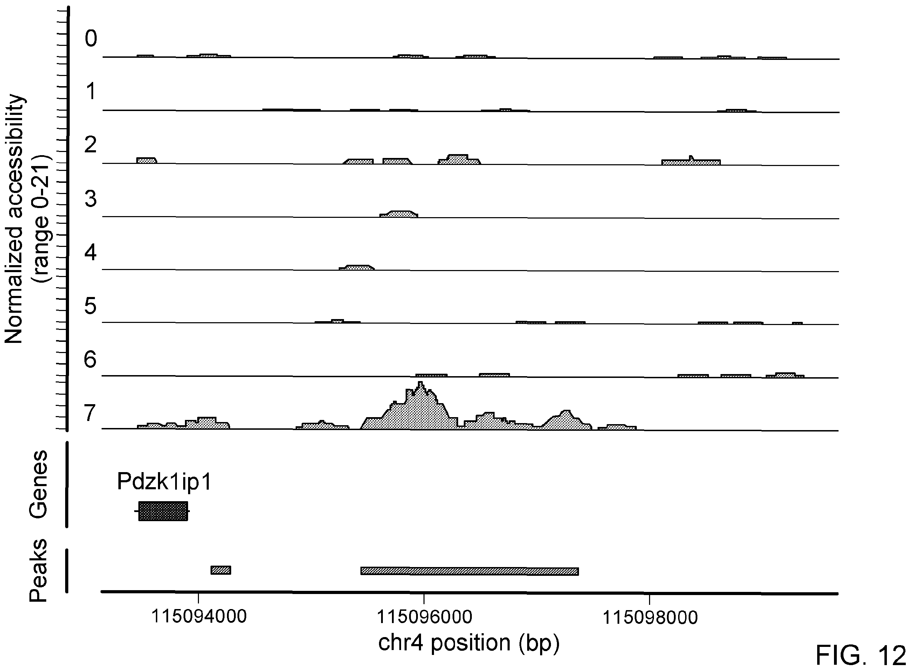

A mouse tissue sample was assayed for genomic region accessibility, where regions were found to be differentially accessible when using the spATAC-seq methods described. **FIG. 12** shows genomic traces of spatial ATAC-seq signal enrichment and accessibility of one of the regions found to be more accessible in a mouse tissue section.

Collectively, the data from **FIGs. 9A-12** demonstrate that the exemplary workflow, including the tissue preparation and spatial ATAC workflow, can be used to detect accessible genomic DNA from biological samples.

### Sequence Listing Appendix

**SEQ ID NO: 1 - Tn5 Transposase** 
MITSALHRAADWAKSVFSSAALGDPRRTARLVNVAAQLAKYSGKSITISSEGSEAMQEG
AYRFIRNPNVSAEAIRKAGAMQTVKLAQEFPELLAIEDTTSLSYRHQVAEELGKLGSIQD
KSRGWWVHSVLLLEATTFRTVGLLHQEWWMRPDDPADADEKESGKWLAAAATSRLR
MGSMMSNVIAVCDREADIHAYLQDKLAHNERFVVRSKHPRKDVESGLYLYDHLKNQP
ELGGYQISIPQKGVVDKRGKRKNRPARKASLSLRSGRITLKQGNITLNAVLAEEINPPKG
ETPLKWLLLTSEPVESLAQALRVIDIYTHRWRIEEFHKAWKTGAGAERQRMEEPDNLER
MVSILSFVAVRLLQLRESFTLPQALRAQGLLKEAEHVESQSAETVLTPDECQLLGYLDK
GKRKRKEKAGSLQWAYMAIARLGGFMDSKRTGIASWGALWEGWEALQSKLDGFLAA
KDLMAQGIKI
**SEQ ID NO: 2 Tn5 Mosaic end sequence**
   CTGTCTCTTA TACACATCT
**SEQ ID NO: 3 - Bacteriophage Mu Transposase** 
MKEWYTAKELLGLAGLPKQATNITRKAQREGWEFRQVAGTKGVSFEFNIKSFPVALRA
EILLQQGRIETSQGYFEIARPTLEAHDYDREALWSKWDNASDSQRRLAEKWLPAVQAA
DEMLNQGISTKTAFATVAGHYQVSASTLRDKYYQVQKFAKPDWAAALVDGRGASRRN
VHKSEFDEDAWQFLIADYLRPEKPAFRKCYERLELAAREHGWSIPSRATAFRRIQQLDE
AMVVACREGEHALMHLIPAQQRTVEHLDAMQWINGDGYLHNVFVRWFNGDVIRPKT
WFWQDVKTRKILGWRCDVSENIDSIRLSFMDVVTRYGIPEDFHITIDNTRGAANKWLTG
GAPNRYRFKVKEDDPKGLFLLMGAKMHWTSVVAGKGWGQAKPVERAFGVGGLEEYV
DKHPALAGAYTGPNPQAKPDNYGDRAVDAELFLKTLAEGVAMFNARTGRETEMCGGK
LSFDDVFEREYARTIVRKPTEEQKRMLLLPAEAVNVSRKGEFALKVGGSLKGAKNVYY
NMALMNAGVKKVVVRFDPQQLHSTVYCYTLDGRFICEAECLAPVAFNDAAAGREYRR
RQKQLKSATKAAIKAQKQMDALEVAELLPQIAEPEAPESRIVGIFRPSGNTERVKNQERD
DEYETERDEYLNHSLDILEQNRRKKAI
**SEQ ID NO: 4 - Mu Transposase Recognition Sequence**
   TGAAGCGGCG CACGAAAAAC GCGAAAG
**SEQ ID NO: 5 - Mu Transposase Recognition Sequence**
   GCGTTTCACG ATAAATGCGA AAA
**SEQ ID NO: 6 - Mu Transposase Recognition Sequence**
   CTGTTTCATT TGAAGCGCGA AAG
**SEQ ID NO: 7 - Mu Transposase Recognition Sequence**
   TGTATTGATT CACTTGAAGT ACGAAAA
**SEQ ID NO: 8 - Mu Transposase Recognition Sequence**
   CCTTAATCAA TGAAACGCGA AAG
**SEQ ID NO: 9 - Mu Transposase Recognition Sequence**
   TTGTTTCATT GAAAATACGA AAA
**SEQ ID NO: 10 - IS4 Family Transposase** 
MTHSDAKLWAQEQFGQAQLKDPRRTQRLISLATSIANQPGVSVAKLPFSPADMEGAYR
FIRNENINAEDIAEAGFQSTVSRANEHKELLALEDTTTLSFPHRSIKEELGHTNQGDRTRA
LHVHSTLLFAPQSQTIVGLIEQQRWSEDITKRGQKHQHATRPYKEKESYKWEQASRRVV
ERLGDKMLDVISVCDREADLFEYLTYKRQHQQRFVVRSMQSRCLEEHAQKLYDYAQA
LPSVETKALTIPQKGGRKARNVKLDVKYGQVTLKAPANKKEHAGIPVYYVGCLEQGTS
KDLAWHLLTSEPINNVDDAMRIIGYYERRWLIEDFHKVWKSEGTDVESLRLQSKDNLER
LSVIYAFVATRLLALRFMKEVDELTKESCEKVLGQKAWKLLWLKLESKTLPKEVPDMG
WAYKNLAKLGGWKDTKRTGRASIKVLWEGWFKLQTILEGYELAMSLDH
**SEQ ID NO: 11- *V. harveyi* Transposase Recognition Sequence**
   CTGTCTCTTGATCACAAGT
**SEQ ID NO: 12- *V. harveyi* Transposase Recognition Sequence**
   AGATGTGATCAAGAGACAG
**SEQ ID NO: 13- *V. harveyi* Transposase Recognition Sequence**
   CTGTCTCTTGATCACATCT

Furthermore, the present invention relates to the following items:
1. A method for determining genomic DNA accessibility, the method comprising:
   (a) a biological sample on an array comprising a plurality of capture probes, wherein a capture probe of the plurality of capture probes comprises: (i) a spatial barcode and (ii) a capture domain;
   (b) contacting a plurality of splint oligonucleotides to the biological sample, wherein a splint oligonucleotide hybridizes to the capture domain;
   (c) contacting a transposome to the biological sample to insert transposon end sequences into accessible genomic DNA, thereby generating fragmented genomic DNA;
   (d) hybridizing the fragmented genomic DNA to the splint oligonucleotide and ligating the fragmented genomic DNA to the capture probe;
   (e) releasing one or more non-ligated transposon end sequences from the ligated fragmented genomic DNA; and
   (f) determining (i) a sequence of the spatial barcode or a complement thereof, and (ii) all or a portion of a sequence of the fragmented genomic DNA, or a complement thereof, and using the determined sequences of (i) and (ii) to determine genomic DNA accessibility in the biological sample.
2. The method of item 1, wherein the array comprises one or more features.
3. The method of item 2, wherein the one or more features comprises a bead.
4. The method of any one of items 1-3, wherein the capture probe further comprises a cleavage domain, one or more functional domains, a unique molecular identifier, or combinations thereof.
5. The method of any one of items 1-4, further comprising an active migration step wherein the fragmented genomic DNA is migrated to the array by applying an electric field.
6. The method of any one of items 1-5, wherein the hybridizing in step (b) comprises hybridizing the splint oligonucleotide, or a portion thereof, to the capture domain, or a portion thereof, of the capture probe.
7. The method of any one of items 1-6, wherein the hybridizing in step (d) comprises hybridizing the splint oligonucleotide, or a portion thereof, to a transposon end sequence or a portion thereof, of a fragmented genomic DNA.
8. The method of any one of items 1-7, wherein the ligating is performed using a DNA ligase.
9. The method of any one of items 1-8, further comprising extending a 3' end of the capture probe using the fragmented genomic DNA as a template.
10. The method of item 9, wherein the extending step is performed using a DNA polymerase having strand displacement activity.
11. The method of any one of items 1-8, wherein the ligating step results in the generation of a DNA molecule.
12. The method of any one of items 1-11, further comprising performing gap filling between the splint oligonucleotide and the fragmented genomic DNA.
13. The method of any one of items 1-12, wherein the transposome comprises a transposase enzyme, and wherein the transposase enzyme is a Tn5 transposase enzyme, a Mu transposase enzyme, a Tn7 transposase enzyme, a *Vibrio* species transposase, or functional derivatives thereof.
14. The method of item 13, wherein the Tn5 transposase enzyme comprises a sequence that is at least 80% identical to SEQ ID NO: 1.
15. The method of any one of items 1-14, wherein the determining in step (f) comprises sequencing (i) the spatial barcode or a complement thereof, and (ii) all or a portion of the sequence of the fragmented genomic DNA or a complement thereof, and further determining the location of the accessible genomic DNA in the biological sample.
16. The method of any one of items 1-15, further comprising imaging the biological sample before or after contacting the biological sample with the array.
17. The method of any one of items 1-16, wherein the releasing in step (d) comprises heating the biological sample.
18. The method of item 17, wherein the heating comprises heating to a temperature of about 65°C to 85°C.
19. The method of item 18, wherein the heating comprises heating to a temperature of about 65°C to about 80°C.
20. The method of item 19, wherein the heating comprises heating to a temperature of about 75°C.
21. The method of any one of items 1-20, further comprising staining the biological sample.
22. The method of item 21, wherein the staining comprises hematoxylin and eosin staining.
23. The method of any one of items 1-22, wherein contacting the transposome to the biological sample is performed under a chemical permeabilization condition, under an enzymatic permeabilization condition, or both.
24. The method of item 23, wherein the chemical permeabilization condition comprises a detergent.
25. The method of item 24, wherein the detergent is one or more of NP-40, Tween-20, Triton X-100, and Digitonin.
26. The method of item 25, wherein the detergent is at a concentration from about 0.001% (v/v) to about 1.0% (v/v).
27. The method of any one of items 1-27, wherein contacting the transposome to the biological sample is performed after an enzymatic pre-permeabilization condition.
28. The method of item 28, wherein the enzymatic pre-permeabilization condition comprises a protease.
29. The method of item 29, wherein the protease is a pepsin, a collagenase, a Proteinase K, and combinations thereof.
30. The method of item 30, wherein the protease is collagenase.
31. A method for determining genomic DNA accessibility, the method comprising:
   (a) a biological sample on an array comprising a plurality of capture probes, wherein a capture probe of the plurality of capture probes comprises: (i) a spatial barcode and (ii) a capture domain;
   (b) contacting a transposome to the biological sample to insert transposon end sequences into accessible genomic DNA, thereby generating fragmented genomic DNA;
   (c) hybridizing a transposon end sequence of the fragmented genomic DNA to the capture domain of the capture probe;
   (d) releasing transposon end sequences not bound to the capture domain; and
   (e) determining (i) a sequence of the spatial barcode or a complement thereof, and (ii) all or a portion of a sequence of the fragmented genomic DNA, or a complement thereof, and using the determined sequences of (i) and (ii) to determine genomic DNA accessibility in the biological sample.
32. The method of item 31, wherein the array comprises one or more features.
33. The method of item 32, wherein the one or more features comprises a bead.
34. The method of any one of items 31-33, wherein the capture probe further comprises a cleavage domain, one or more functional domains, a unique molecular identifier, or combinations thereof.
35. The method of any one of items 31-34, further comprising an active migration step wherein the fragmented genomic DNA is migrated to the array by applying an electric field.
36. The method of any one of items 31-35, wherein the hybridizing in step (c) comprises hybridizing the transposon end sequence, or a portion thereof, to the capture domain, or a portion thereof, of the capture probe.
37. The method of any one of items 31-36, further comprising extending a 3' end of the capture probe using the fragmented genomic DNA as a template.
38. The method of item 37, wherein the extending step is performed using a DNA polymerase having strand displacement activity.
39. The method of any one of items 31-38, further comprising performing gap filling between the transposon end sequence and the fragmented genomic DNA.
40. The method of any one of items 31-39, wherein the transposome comprises a transposase enzyme, and wherein the transposase enzyme is a Tn5 transposase enzyme, a Mu transposase enzyme, a Tn7 transposase enzyme, a *Vibrio* species transposase, or functional derivatives thereof.
41. The method of item 40, wherein the Tn5 transposase enzyme comprises a sequence that is at least 80% identical to SEQ ID NO: 1.
42. The method of any one of items 31-41, wherein the determining in step (e) comprises sequencing (i) the sequence of the spatial barcode or a complement thereof, and (ii) all or a portion of the sequence of the fragmented genomic DNA or a complement thereof and further determining the location of the accessible genomic DNA in the biological sample.
43. The method of any one of items 31-42, further comprising imaging the biological sample before or after contacting the biological sample with the array.
44. The method of any one of items 31-43, wherein the releasing in step (d) comprises heating the biological sample.
45. The method of item 44, wherein the heating comprises heating to a temperature of about 65°C to 85°C.
46. The method of item 45, wherein the heating comprises heating to a temperature of about 65°C to about 80°C.
47. The method of item 46, wherein the heating comprises heating to a temperature of about 75°C.
48. The method of any one of items 31-47, further comprising staining the biological sample.
49. The method of item 48, wherein the staining comprises hematoxylin and eosin staining.
50. The method of any one of items 31-49, wherein contacting the transposome to the biological sample is performed after a chemical permeabilization condition, under an enzymatic permeabilization condition, or both.
51. The method of item 50, wherein the chemical permeabilization condition comprises a detergent.
52. The method of item 51, wherein the detergent is one or more of NP-40, Tween-20, Triton X-100, and Digitonin.
53. The method of item 52, wherein the detergent is at a concentration from about 0.001% (v/v) to about 0.1% (v/v).
54. The method of items 31-53, wherein contacting the transposome to the biological sample is performed after an enzymatic pre-permeabilization condition.
55. The method of item 54, wherein the enzymatic pre-permeabilization condition comprises a protease.
56. The method of item 55, wherein the protease is a pepsin, a collagenase, a Proteinase K, and combinations thereof.
57. The method of item 56, wherein the protease in a collagenase.
58. A method for determining the location of DNA in a biological sample, the method comprising:
   (a) a biological sample on an array comprising a plurality of capture probes, wherein a capture probe of the plurality of capture probes comprises: (i) a spatial barcode and (ii) a capture domain;
   (b) contacting the biological sample with a protease, wherein the protease is capable of degrading one or more histone proteins, thereby releasing the DNA;
   (c) contacting a transposome to the biological sample to insert transposon end sequences into the released genomic DNA, thereby generated fragmented genomic DNA;
   (d) hybridizing a transposon end sequence of the fragmented DNA to the capture domain;
   (e) releasing transposon end sequences not bound to the capture domain; and
   (f) determining (i) a sequence of the spatial barcode or a complement thereof, and (ii) all or a portion of a sequence of the DNA, or a complement thereof, and using the determined sequences of (i) and (ii) to determine the location of DNA in the biological sample.
59. The method of item 58, wherein the protease is capable of degrading at least one linker histone protein and at least one core histone protein in the biological sample.
60. The method of item 58 or 59, wherein the protease is capable of degrading at least one histone from each core histone family in the biological sample.
61. The method of any one of items 58-60, wherein the protease is a serine protease, an aspartyl protease, a peptidase family C1 enzyme, a protease that is inhibited by the diazomethane inhibitor Z-Phe-Phe-CHN(2) or the epoxide inhibitor E-64, a lysosomal protease, collagenase, or an azurophilic enzyme.
62. The method of item 61, wherein the protease is collagenase.
63. The method of any one of items 58-62, wherein the capture domain comprises a homopolymeric sequence.
64. The method of any one of items 58-62, wherein the capture domain comprises a unique sequence.
65. The method of any one of items 58-64, wherein the capture probe further comprises a cleavage domain, one or more functional domain, a unique molecular identifier, or combinations thereof.
66. The method of any one of items 58-65, further comprising an active migration step wherein the fragmented genomic DNA is migrated to the array by applying an electric field.
67. The method of any one of items 58-66, wherein the hybridizing in step (d) comprises hybridizing the transposon end sequence, or a portion thereof, to the capture domain, or a portion thereof, of the capture probe.
68. The method of any one of items 58-67, further comprising extending a 3' end of the capture probe using the fragmented genomic DNA as a template.
69. The method of item 68, wherein the extending step is performed using a DNA polymerase having strand displacement activity.
70. The method of any one of items 58-69, further comprising gap filling between the transposon end sequence and the fragmented genomic DNA.
71. The method of any one of items 58-70, wherein the transposome comprises a transposase enzyme, and wherein the transposase enzyme is a Tn5 transposase enzyme, a Mu transposase enzyme, a Tn7 transposase enzyme, a Vibrio species transposase, or functional derivatives thereof.
72. The method of item 71, wherein the Tn5 transposase enzyme comprise a sequence that is at least 80% identical to SEQ ID NO: 1.
73. The method of any one of items 58-72, wherein the determining in step (f) comprises sequencing (i) the spatial barcode or a complement thereof, and (ii) all or a portion of the sequence of the fragmented genomic DNA or a complement thereof.
74. The method of any one of items 58-73, wherein the method further comprises imaging and/or staining the biological sample.
75. The method of item 74, wherein the staining comprises haematoxylin and eosin staining.
76. The method of any one of items 58-75, wherein the protease is contacted with the biological sample from about 5 minutes to about 15 minutes.
77. The method of item 76, wherein the protease is contacted with the biological sample for about 10 minutes.
78. The method of any one of items 58-77, wherein the protease is contacted with the biological sample at a temperature from about 30°C to about 45°C.
79. The method of item 78, wherein the protease is contacted with the biological sample at a temperature of about 37°C.
80. The method of any one of items 58-79, wherein the releasing in step (d) comprises heating the biological sample.
81. The method of item 80, wherein the heating comprises heating to a temperature of about 65°C to 85°C.
82. The method of any of items 58-81, wherein determining the location of DNA in a biological sample further comprises spatially analyzing the whole genome of the biological sample.
83. The method of any one of items 1-82, wherein the biological sample is a tissue section.
84. The method of item 83, wherein the tissue section is a fresh, frozen tissue section.
85. The method of item 83, wherein the tissue section is a fixed tissue section.
86. The method of item 85, wherein the fixed tissue section is a formalin-fixed paraffin-embedded fixed tissue section, an acetone fixed tissue section, a paraformaldehyde fixed tissue section, or a methanol fixed tissue section.

## Claims

1. A method for determining genomic DNA accessibility, the method comprising:
(a) providing a biological sample on an array comprising a plurality of capture probes, wherein a capture probe of the plurality of capture probes comprises: (i) a spatial barcode and (ii) a capture domain;
(b) contacting the biological sample with a protease to degrade one or more histone proteins from the biological sample, thereby releasing accessible genomic DNA from the one or more histone proteins;
(c) contacting the biological sample with a transposome to insert a transposon end sequence into the released accessible genomic DNA, thereby generating fragmented genomic DNA;
(d) hybridizing the transposon end sequence of the fragmented genomic DNA to the capture probe;
(e) releasing transposon end sequences not bound to the capture domain; and
(f) determining (i) a sequence of the spatial barcode or a complement thereof, and (ii) all or a portion of a sequence of the fragmented genomic DNA, or a complement thereof, and using the determined sequences of (i) and (ii) to determine the location of genomic DNA accessibility in the biological sample.

2. The method of claim 1, wherein the protease is capable of degrading at least one linker histone protein and at least one core histone protein in the biological sample; optionally, wherein the protease is capable of degrading at least one histone from each core histone family in the biological sample.

3. The method of claim 1 or 2, wherein the protease is a serine protease, an aspartyl protease, a peptidase family Cl enzyme, a protease that is inhibited by the diazomethane inhibitor Z-Phe-Phe-CHN(2) or the epoxide inhibitor E-64, a lysosomal protease, collagenase, or an azurophilic enzyme; optionally, wherein the protease is collagenase.

4. The method of any one of claims 1-3, wherein the capture probe comprises a splint oligonucleotide, wherein the splint oligonucleotide hybridizes to the capture domain of the capture probe and the transposon end sequence of the fragmented genomic DNA.

5. The method of any one of claims 1-4, wherein the capture probe further comprises a cleavage domain, one or more functional domains, a unique molecular identifier, or combinations thereof.

6. The method of any one of claims 1-5, wherein the hybridizing in (d) comprises hybridizing the transposon end sequence, or a portion thereof, to the capture probe.

7. The method of any one of claims 1-6, further comprising extending a 3' end of the capture probe using the fragmented genomic DNA as a template.

8. The method of claim 7, wherein the extending is performed using a DNA polymerase having strand displacement activity.

9. The method of any one of claims 1-8, further comprising gap filling between the transposon end sequence and the fragmented genomic DNA.

10. The method of any one of claims 1-9, wherein the transposome comprises a transposase enzyme, and wherein the transposase enzyme is a Tn5 transposase enzyme, a Mu transposase enzyme, a Tn7 transposase enzyme, a Vibrio species transposase, or functional derivatives thereof; optionally, wherein the Tn5 transposase enzyme comprise a sequence that is at least 80% identical to SEQ ID NO: 1.

11. The method of any one of claims 1-10, wherein the determining in (f) comprises sequencing (i) the sequence of the spatial barcode or a complement thereof, and (ii) all or a portion of the sequence of the fragmented genomic DNA or a complement thereof.

12. The method of any one of claims 1-11, wherein the method further comprises imaging and/or staining the biological sample; optionally, wherein the staining comprises hematoxylin and eosin staining.

13. The method of any one of claims 1-12, wherein the protease is contacted with the biological sample from about 5 minutes to about 15 minutes; optionally about 10 minutes.

14. The method of any one of claims 1-13, wherein the protease is contacted with the biological sample at a temperature from about 30°C to about 45°C, optionally at a temperature of about 37°C.

15. The method of any one of claims 1-14, wherein the releasing in (e) comprises heating the biological sample; optionally to a temperature of about 65°C to 85°C.

16. The method of any one of claims 1-15, wherein the biological sample is a tissue section, optionally a fresh, frozen tissue section, a fixed tissue section, a formalin-fixed paraffin-embedded fixed tissue section, an acetone fixed tissue section, a paraformaldehyde fixed tissue section, or a methanol fixed tissue section.

17. The method of any one of claims 1-16, wherein the providing the biological sample on the array in (a) comprises aligning the biological sample with the array prior to transferring the accessible genomic DNA from the biological sample to the array.
